# EUROPEAN PATENT SPECIFICATION

(11) **EP 2 470 081 B1**
(45) Date of publication and mention of the grant of the patent: **06.06.2018**
(21) Application number: 10814053.4
(22) Date of filing: 18.08.2010
(51) Int. Cl.: A61B 17/00

(54) **SPINAL IMPLANT**
WIRBELSÄULENIMPLANTAT
IMPLANT SPINAL

(30) Priority: 27.08.2009 US 583865; 30.07.2010 US 804867; 27.08.2009 US 583864
(43) Date of publication of application: 04.07.2012
(73) Proprietor: K2M, Inc., Leesburg, VA 20175 (US)
(72) Inventor: CASTRO, Frank, Louisville KY 40245 (US)
(74) Representative: Siekmann, Gunnar
(86) International application number: PCT/US2010/002269
(87) International publication number: WO 2011/028236

(56) References cited:
- US-A- 5 558 674
- US-A- 6 066 175
- US-A- 6 159 245
- US-A1- 2007 016 295
- US-A1- 2007 123 987
- US-A1- 2007 129 805
- US-A1- 2007 255 413
- US-A1- 2008 015 694
- US-A1- 2008 021 476
- US-B1- 7 435 261
- US-B2- 6 660 038
- US-B2- 6 776 798
- US-B2- 6 808 538
- US-B2- 6 863 673
- US-B2- 6 926 737
- US-B2- 7 182 782
- US-B2- 7 232 463
- US-S1- D 524 942

## Description

### BACKGROUND OF THE INVENTION

### 1. Field of the Invention

Among other things, the present invention is related to a surgical implant or cage that can be inserted into a cavity that has been created by removing spinal tissue. The cage includes a series of trapezoidal dividers or braces, first and second universal corner posts and lateral universal posts manufactured in such a way that the surgeon can see through the cage's openings prior to the addition of substances. Preferred embodiments allow the surgeon to view the dura mater and other spinal tissues prior to the insertion of osteogenic and other substances.

In select preferred embodiments, the spinal implant includes a generally horizontal divider, a first set of dividers diverging away from the horizontal and a second set of dividers diverging away from the horizontal, where the dividers are positioned on either side of the generally horizontal divider. One or more brakes can be incorporated into the spinal implant, and the brakes can include bores for receiving fasteners. In some preferred embodiments, first and second lateral brakes are integral with the spinal implant. Still other select preferred embodiments utilize one or more brakes that are connectable to the lateral supports of the spinal implant. In select preferred embodiments, the spinal implant includes one or more laterally extending grips that can also function as brakes. The grip members can include bores for receiving fasteners.

Other preferred embodiments of the current spinal implant are provided with superior and inferior plates having bores that can receive fasteners. Still other select preferred embodiments include one or more bars generally parallel to one or more of the outward sides of the series of dividers. And still other preferred embodiments can employ one or more end caps to assist with the stabilization of the spinal implant in the surgically created cavity.

### B. Description of the Previous Art

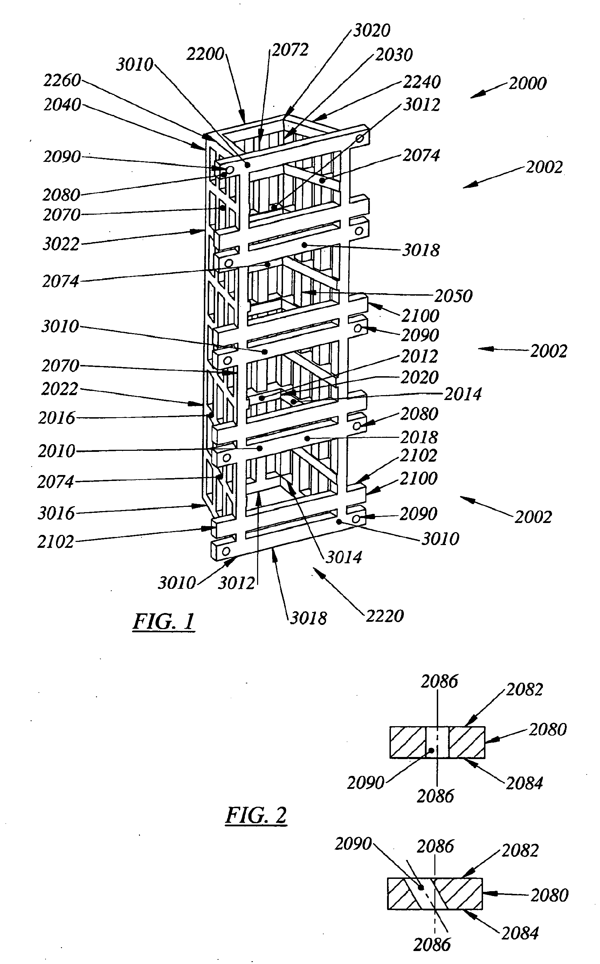

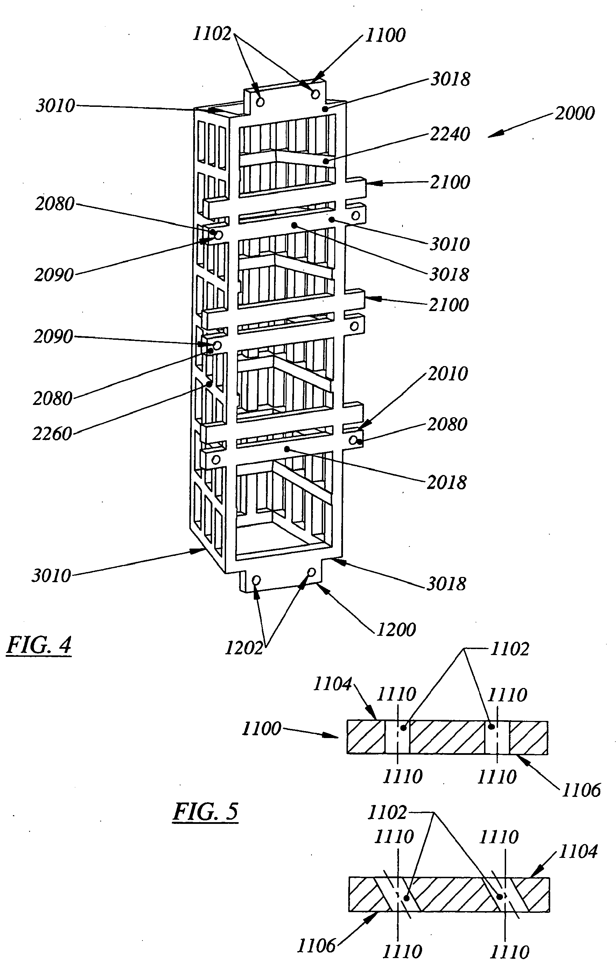

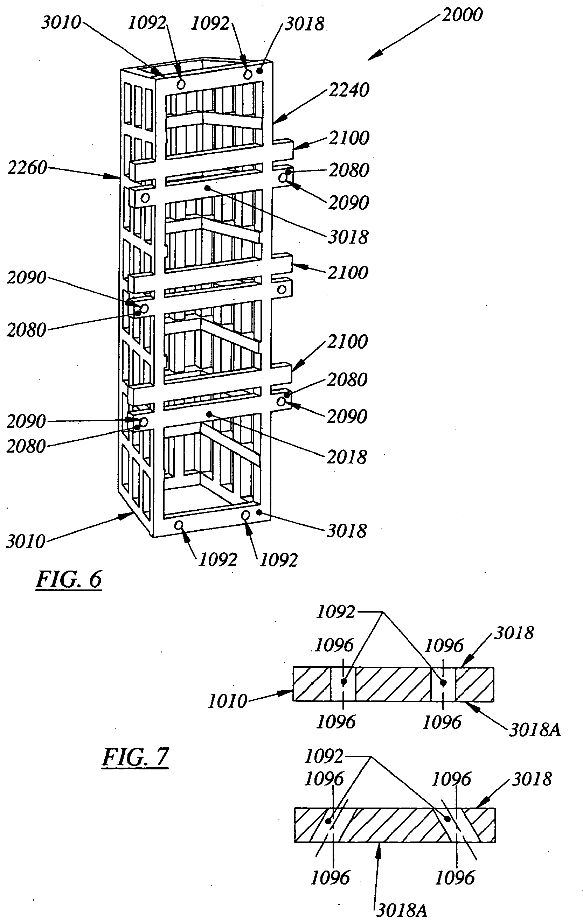

Any discussion of references cited in this Description of the Previous Art merely summarizes the disclosures of the cited references and Applicant makes no admission that any cited reference or portion thereof is relevant prior art. Applicant reserves the right to challenge the accuracy, relevancy and veracity of the cited references.
1) US Published Patent Application No. 20030125739 A1-Bagga, et al. discloses a bioactive spinal implant and method of manufacture. Among other things, it does not appear that the Bagga invention practices the use of a series of dividers, universal corner posts, universal posts, bars, laterally extending grips, connectable brakes or end caps.
2) US Patent No. 6,767,367 B1-Michelson enables a spinal fusion implant having deployable bone engaging projections. Michelson teaches that the' 367 implant 300 has a rotatable member 320 that is preferably frustoconical in shape. Rotatable member 320 has bone engaging projections 332 adapted to penetrably engage the bone of the adjacent vertebral bodies. Bone engaging projections 332 are preferably configured such that in a retracted position, implant 300 may be linearly inserted into the disc space. After implant 300 is inserted into the disc space, bone engaging projections 332 are moved to a deployed position to penetrably engage the endplates of each adjacent vertebral body and prevent the expulsion of implant 300 from the disc space.
3) US Patent No. 6,537,320 B1-Michelson enables a self-broaching, rotable, push-in interbody spinal fusion implant and method for its deployment. Among other things, it does not appear that the Michelson invention practices the use of a series of dividers, universal corner posts, universal posts, grips, bars connectable brakes or end caps.
4) US Patent No. 5,609,635-Michelson enables a lordotic interbody spinal fusion implant. The Michelson Summary of the Invention teaches, "In the preferred embodiment of the modular implant, the implant is again wedge-shaped in the side elevational view and is taller at its insertion end than at its trailing end." Being taller at an insertion end than at the trailing end is a teaching that is incompatible with the current spinal implant. Further, among other things, it does not appear that the Michelson invention practices the use of a series of dividers, universal corner posts, universal posts, grips, bars, connectable brakes or end caps.
5) US Patent No. 6,302,914 B1-Michelson enables a lordotic interbody spinal fusion implant. The '914 Patent is a continuation Patent of the 5,609,635-Michelson Patent and enables the same limitations as the '635 Patent.
6) US Patent No. 6,066,175-Henderson, et al. enables a fusion stabilization chamber. Henderson discloses a mesh cage 41, barrel vaults 48 and 49 and flanges 50 and 51 to anchor the cage. The cage is preferably rectangular when viewed from the top or the bottom. Among other things, it does not appear that the Henderson invention practices the use of a series of dividers, universal corner posts, universal posts, bars, connectable brakes or end caps.
7) US Patent 5,766,252-Henry, et al. enables an interbody spinal prosthetic implant and method. Henry requires a threaded hole 38 to practice the '252 invention. Among other things, it does not appear that the Henry invention practices the use of a series of dividers, universal corner posts, universal posts, bars, grips, connectable brakes and end caps.
8) US Patent 5,425,772-Brantigan enables a prosthetic implant for intervertibral spinal fusion. The '772 device practices traverse teeth or serrations 19 where the teeth have sharp peaks 19*a*, slopping walls 19*b* and valleys 19*c*. Among other things, it does not appear that the Brantigan invention practices the use of a series of dividers, universal corner posts, universal posts, bars, grips, connectable brakes or end caps.
9) US Patent No. 5,147,402-Bohler, et al. enables an implant for ingrowth of osseous tissue. Among other things, it does not appear that the Bohler invention practices the use of a series of dividers, universal corner posts, universal posts, bars, grips, connectable brakes or end caps.
10) US Patent No. 6,746,484-Liu, et al. enables a spinal implant. Among other things, Liu teaches an implant 1 having an elongate hollow body 3, parallel longitudinal walls 4 and terminal ends 5 and 6 including threaded holes 11 *a*. Among other things, it does not appear that the Liu invention practices the use of a series of dividers, universal corner posts, universal posts, bars, grips, connectable brakes or end caps.
11) US Patent No. 6,231,610 B1-Geisler enables an anterior cervical column support device. The '610 apparatus utilizes serrations on the load bearing surfaces and two screw holes. Among other things, it does not appear that the Geisler invention practices the use of a series of dividers, universal corner posts, universal posts or bars. Among other things, it does not appear that the Geisler invention practices the use of a series of dividers, universal corner posts, universal posts, grips, bars, connectable brakes and end caps.
12) US Patent No. 6,660,038 B2-Boyer, et al. enables skeletal reconstruction cages. The Boyer Patent discloses an end cap 210 suitable for coupling to central shaft 160 includes an outer wall 212, as well as a central hole disposed along axis 213 with a lower inner wall 214, an upper inner wall 216, and an inner ridge portion 218. Notably, while outer wall 176 of central shaft 160 is generally circular, outer wall 212 of end cap 210 is generally oblong, so that a generally I-shaped skeletal reconstruction cage may be formed when a pair of end caps 210 are placed on central shaft 160. Among other things, it does not appear that the Boyer invention practices the use of a series of dividers, universal corner posts, universal posts, bars, grips and connectable brakes.
13) US Patent No. 6,491,724-Ferree enables a spinal fusion cage with lordosis correction. Among other things, Ferree teaches an anterior portion 112 that includes mating members 120 and 122 with teeth 124 or other features to form a locking or ratchet mechanism that is used to alter the height of the cage. Among other things, it does not appear that the Ferree invention practices the use of a series of dividers, universal corner posts, universal posts, bars, grips, connectable brakes or end caps.
14) US Patent No. 6,117,174-Nolan enables a spinal implant device that has disc 14 made of the same material as body 12. Among other things, it does not appear that the Nolan invention practices the use of a series of dividers, universal corner posts, universal posts, bars, grips, connectable brakes or end caps.
15) US Published Patent Application No. 20070016295-Boyd discloses a reinforced molded implant formed of cortical bone. Boyd reads, "Implant 10 defines a longitudinal axis 11 and includes a first strut 12, a second strut 14 spaced from first strut 12, and cross-member 16 extending therebetween. First strut 12 and second strut 14 are each positioned to lie in a plane substantially parallel to longitudinal axis 11. Implant 10 includes at least one additional cross-member 16 A connecting first strut 12 and second strut 14. It is understood that in alternative embodiments implant 10 can have one or a plurality of cross-members connecting first strut 12 to second strut 14."
16) US Patent No. 6,090,143-Meriwether, et al. enables a box cage for intervertebral body fusion. Meriwether reads, "FIG. 6 illustrates a further embodiment of the invention which is a slight modification of that shown in FIG. 4. In the embodiment of FIG. 6, rather than having a rectangular longitudinal cross-section, it is trapezoidal such that the resulting cage member, indicated generally by numeral 110, is wedge-shaped. The assembled cage comprises a box-like base 112 and a cover 114 dimensioned to fit over the base much like the cover on a shirt box. The height dimension of the rightmost ends of the base and cover are greater than the height dimension of the corresponding, opposed left side ends, thus providing the desired wedge shape. Upwardly projecting ribs 116 and 118 extend along the rear and front side edges, respectfully, and likewise, the base 112 includes longitudinally extending ribs 120 and 122 projecting downwardly from the undersurface of the base along the side edges thereof. The right and left ends of the base 112 and the cover 114 include semi-circular cut-outs as at 124 and 126 and 128-130 such that when the cover 114 is placed upon the base 112, circular apertures are formed. These apertures are adapted to receive a tapered screw 132 therein. The slope of the taper of the screw is designed to correspond to that of the cage assembly 110 such that when the screw 132 is threaded into the circular opening defined by arcuate cut-outs 124 and 126 and advanced by turning until the leading end 134 of the screw enters the circular aperture 128-130, further turning of the screw will raise the case cover 114 relative to its base 112, allowing adjustment of the cage height following positioning thereof between adjacent vertebral bodies."
17) US Patent No. 6,159,245-Meriwether, et al. enables a box cage for intervertebral body fusion. The '245 Meriwether Patent is a continuation of US Patent No. 6,090,143-Meriwether, et al. and teaches the same limitations as the '143 Patent.
18) US Patent No. 6,432,107-Ferree enables enhanced surface area spinal fusion devices. The '107 Patent teaches, "The device 200 fits into slots 204 and 206 made in upper and lower vertebrae 208 and 210 , respectively, allowing the lower section to fuse within the body of the lower vertebrae 210, and the upper section to fuse within the body of the upper vertebrae 208 . Thus, in contrast to existing devices, the device 200 and the alternative embodiments disclosed herein feature considerably more intimate contact with cancellous bone due to the fact that the device is inserted directly into the cavities 204 and 206. Rather than a relatively minor amount of scraping of the end plates of the vertebrae to be distracted, the entire end portions of the device 200 which penetrate the upper and lower vertebrae make contact with cancellous bone, thereby enhancing fusion considerably. FIG. 2B is a cross-section of a vertebrae of FIG. 2A as viewed from a top-down perspective, showing how the device fits tightly along the entire walls of the channels created in the vertebrae." Among other things, the Ferree device does provide a series of dividers or receptacles visible to the surgeon after insertion into the surgical cavity, grips, connectable brakes or end caps.
19) US Patent No. 6,569,201-Moumene, et al. enables a hybrid composite interbody fusion device. Moumene's Osteoinductive pore 2 forms a void 23 within the support and defines an inner surface 13, and opens onto side surface openings 19, 21 formed in side surfaces 7, 9, and the void section 23 is suitable for housing a bone growth material such as bone chips (not shown). Among other things, it does not appear that the Moumene invention practices the use of a series of dividers, universal corner posts, universal posts, grips, bars, connectable brakes or end caps.
20) US Patent No. 7,435,261 - Castro discloses a spinal implant and a method for using spinal implant. The spinal implant comprises some generally trapezoidal shaped dividers and several supports. The dividers are connected to each support in defined distances.

### SUMMARY OF THE INVENTION

Unlike traditional spinal implants, the present invention is a spinal implant as defined in the independent claim and dependent claims that, among other things, can be packed with bone graft and/or other substances before and/or after the spinal implant has been inserted into a cavity created by the removal of spinal tissue. Through openings in the spinal implant, prior to insertion of osteogenic or other substances, the surgeon can view the dura mater, other spinal tissue as well as other tissues. During surgical procedures, the wedge-like cage assists the surgical team in not impinging the spinal cord with the implant. Post operative and prior to complete arthrodesis, the generally wedge-like implant inhibits extrusion of the cage against the spinal cord. Select preferred embodiments employ bars, bores, brakes, end caps, grips, inferior dividers, superior dividers, inferior plates and superior plates to assist with the stabilization of the spinal implant.

An aspect of the present invention is to provide a generally wedge-like cage.

Still another aspect of the present invention is to provide a spinal implant having a series of trapezoidal dividers or braces, first and second universal corner posts and lateral universal posts.

It is still another aspect of the present invention to provide a spinal implant that can be implanted through the patient's frontal or rearward side. An example not forming part of the present invention comprises a cage that can be severed across a first cross-section or severed across a first cross-section and a second cross-section to create a custom fitted implant for the surgically created cavity. An example not forming part of the present invention comprises a cage including a generally straight brace, a first set of braces diverging away from the horizontal and a second set of braces diverging away from the horizontal. An example not forming part of the present invention comprises an implant having a plurality of apertures the surgeon can see through prior to addition of osteogenic, arthrodesis accelerating substances and/or other substances. An example not forming part of the present invention comprises one or more bars proximate one or more outward sides of the wedge-like cage's series of braces. An example not forming part of the present invention comprises an implant that includes one or more brakes having bores for receiving fasteners such as bone screws. An example not forming part of the present invention comprises an implant that includes upper and lower plates having one or more bores for receiving fasteners.

Still another aspect of the present invention is to provide a cage including one or more laterally extending grips that can also function as brakes.

Another aspect of the present invention is to provide an implant that includes grips with one or more members having bores for receiving fasteners such as bone screws. An example not forming part of the present invention comprises a brake that is manufactured separately from the spinal implant, where the brake is connectable to the spinal implant. An example not forming part of the present invention comprises a connectable brake that includes one or more bores for receiving fasteners.

Yet another aspect of the present invention is to provide an end cap attachable to vertebra for assisting with the stabilization of the spinal implant. An example not forming part of the present invention comprises an end cap capable of increasing the length of the spinal implant. An example not forming part of the present invention comprises a spinal implant capable of enhancing lordosis.

Among other things, it is believed that one or more aspects of the present invention decrease the possibility of spinal cord injury and increase the probability of bony fusion when compared to prior spinal implants. Because the spinal implant's structural geometry improves resistance against gravitation forces, conforms to the volume of the surgically created cavity and includes openings at its lengthwise ends capable of contacting healthy vertebral tissue, bony fusion and structural stability of surgical cavity are improved.

Compared to US Patent No. 7,435,261 - Castro the invention according to claim 1 aims to provide a cage, which facilitates an improved handling, particularly as it facilitates a better grip of the cage and a more tight fitting of the cage in the cavity of the spinal cord and advantageously provides the cage from impinging the spinal cord.

An embodiment of the present apparatus can be described as a cage as defined in the independent claim and dependent claims. It is the novel and unique interaction of these simple elements which creates the spinal implants, within the ambit of the present invention. Descriptions of preferred embodiments follow. However, it is to be understood that the best mode descriptions do not limit the scope of the present invention.

### BRIEF DESCRIPTION OF THE DRAWINGS

FIG. 1 is a perspective of cage (2000) having a series of trapezoidal dividers (2010) and (3010) and a plurality of receptacles (2002).
FIG. 2 shows bore (2090) of brake (2080).
FIG. 3 is a lateral view of second lateral support (2260) of implant (2000).
FIG. 4 is a perspective of cage (2000) with superior plate (1 100) that extends upward from divider (3010) and an inferior plate (1200) that depends downward from inferior divider (3010).
FIG. 5 shows bore (1 102) of superior plate (1 100).
FIG. 6 is a perspective of implant (2000) with superior and inferior braces (3010) and bores (1092).
FIG.7 shows bore (1092) of superior and inferior braces (3010).
FIG. 8 is a perspective of cage (2000) having a series of trapezoidal dividers (2010) and a plurality of receptacles (2002).
FIG. 9 shows bore (2090) of brake (2080).
FIG. 10 is a perspective of spinal implant (2000) including bars (2085) and lateral extensions (2082).
FIG. 11 is a perspective of cage (2000) with superior plate (1100) that extends upward from divider (2010) and an inferior plate (1200) that depends downward from inferior divider (2010).
FIG. 12 shows bore (1102) of superior plate (1100).
FIG. 13 is a perspective of spinal implant (2000) having superior and inferior braces (2010) including bores (1092).
FIG. 14 shows bore (1092) of superior and inferior braces (2010).
FIG. 15 is a perspective of cage (2000) with first lateral brake (3200) and second lateral brake (3300).
FIG. 16 shows bore (3260) of first lateral brake (3200) or second lateral brake (3300).
FIG. 17 is a perspective of cage (2000) with superior plate (1100) that extends upward from divider (3010) and an inferior plate (1200) that depends downward from inferior divider (3010).
FIG. 18 shows bore (1102) of superior plate (1100).
FIG. 19 is a perspective of spinal implant (2000) having superior and inferior braces (2010) including bores (1092).
FIG. 20 shows bore (1092) of superior and inferior braces (2010).
FIG. 21 is a lateral view of second lateral support (2260) of implant (2000).
FIG. 22 is an exploded view of cage (2000) that utilizes a pair of connectable lateral brakes (3500).
FIG. 23 shows bore (1092) of superior and inferior braces (2010).
FIG. 24 is a perspective of inward side (3504) of a connectable lateral brake (3500).
FIG. 25 is a frontal view of edge (3508) of connectable lateral brake (3500).
FIG. 26 is an exploded frontal perspective of end cap (3900).
FIG. 27 is a perspective of end cap (3900) and implant proximate surface (3924) opposite implant distal surface (3922) of outermost section (3912) of bridge (3910).
FIG. 28 is a perspective view of bridge facing side of coupler (3950).
FIG. 29 is an exploded view of a preferred embodiment of an end cap (3900) and cage (2000).
FIG. 30 is an exploded view of a preferred embodiment of an end cap (3900) and cage (2000).
FIG. 31 is an exploded view of cage (2000) and a plurality of attachable lateral brakes (3600).

### DESCRIPTION OF THE PREFERRED EMBODIMENTS

Although the disclosure hereof is detailed to enable those skilled in the art to practice the invention, the embodiments published herein merely exemplify the present invention.

In the most general sense, the present invention is a cage or implant that can be inserted into a cavity of the spinal column. Surgical removal of mammalian spinal tissue in one or more spinal regions creates the cavity or cavities that will receive the implant or implants. It has been discovered that many embodiments of the current implant can be useful for cervical spine surgeries and are capable of assisting with the stabilization of the postoperative spine. And many of the preferred embodiments of the present invention are particularly suited for corpectomy or partial corpectomy procedures. However, other preferred embodiments of the current invention can be utilized for other spinal surgical procedures.

After insertion of the implant into the cavity, the cage assists in stabilizing the spinal column against rotational movement and also resists the compression forces associated with gravity on the spinal column. Select preferred embodiments of the present invention can be implanted through the patient's anterior side, e.g., the frontal side of the patient's neck. The current spinal implants are custom fitted for the surgical cavity into which they will be inserted, i.e., the cage can be manufactured to fit the surgical cavity or the cage can be severed across a first cross section or the cage can be severed across first and second cross sections to size the cage to fit the cavity.

For preferred embodiments of the current spinal implant, universal corner posts and lateral universal posts are spaced about the outer border of the cage. Select preferred embodiments include one or more bars proximate to and parallel with one or more outward sides of the series of dividers. Still other preferred embodiments utilize one or more end caps with the spinal implant. In other preferred embodiments, one or more ties can extend about the implant and contact the universal posts and the universal corner posts.

In select preferred embodiments, universal corner posts and universal posts are from about one millimeter to about two millimeters wide and are situated about the outer periphery of the cage in such a way as to create apertures between the universal lateral posts and the universal corner posts. Depending on the volume of the implant, spacing between the universal corner posts and the lateral universal posts and between the lateral universal posts is from about one millimeter to about two millimeters.

Preferred embodiments of the current spinal implant have one more trapezoidal or trapezoidal-like dividers or braces. Cages are manufactured of titanium alloys, stainless steel, resorbable polymers, non-resorbable polymers or any other composition acceptable in the art. It has advantageously been discovered that the current cages can have a width of from about six to about fifteen millimeters, as measured along the narrowest parallel of the trapezoid, and a depth of from about eight millimeters to about fifteen millimeters, as measured along a converging side of the trapezoid. In select preferred embodiments, the spacing between dividers of the cage is approximately twelve millimeters, as measured from outward side to outward side of the series of dividers. Openings of the sides of the cages of the current invention into which bone graft, osteogenic and/or arthrodesis accelerating substances are packed can have areas from about 36 millimeters² to 225 millimeters² or greater.

Preferred embodiments of the present invention are provided with one or more brakes to further impede the implant from contacting the spinal column. Brakes can include one or more bores for receiving fasteners, such as screws. Other select preferred embodiments are provided with first and/or second lateral brakes that extend from approximate the superior edge to approximate the inferior edge of the spinal implant. Still other preferred embodiments utilize one or more lateral brakes that are connectable with the spinal implant.

Select preferred embodiments include one or more grips. Among other things, the grips can also function as brakes. One or more of the grip members can also be provided with a bore for receiving a fastener.

Meeting a long felt but unfilled need in the spinal surgical arts, the unique structures of the present invention allow the surgical team to view the dura mater, before the cage is packed. Allowing the surgical team to view the dura mater while inserting the implant into the cavity reduces the possibility of having the cage inadvertently contact or injure the spinal cord. Meeting another long felt but unfilled need, the current spinal implant's wedge-like cage increases contact between the implant and tissue surrounding the surgically created cavity. And it is believed that the increased contact enhances post operative spinal stabilization resulting in enhanced spinal rigidity that improves bony fusion. Testing has also revealed that the cage's wedge-like structure is capable of withstanding greater compressive loads than prior art round or oval implants.

Contact between the surgical cavity wall and the novel and unobvious structures of the spinal implant can also inhibit the implant from contacting the spinal cord, thereby reducing the possibility of spinal cord injury as compare to prior spinal implants. It appears that having the apertures of select embodiments in such close proximity with the cavity's walls increases the probability of the osteogenic materials procuring a blood supply. And it is believed that increasing the blood supply to the osteogenic materials held by the cage enhances local areas of arthrodesis between the vertebra and the bone graft.

Among other things, FIG. 1 enables a cage (2000) having a series of trapezoidal dividers (2010) and (3010) and a plurality of receptacles (2002). Generally horizontal divider or brace (2010) has inward side (2012) (after insertion into the surgically created cavity, positioned near the dura mater of the spinal cord), outward side (2018) (proximate the surgeon after insertion into the surgically created cavity), first converging or lateral side (2014) and second converging or lateral side (2016). Intersection of first lateral side (2014) and inward side (2012) creates first corner (2020) and intersection of inward side (2012) and second lateral side (2016) creates second corner (2022), such that straight brace (2010) has first corner (2020) and second corner (2022).

A first set of braces or dividers (3010) is positioned in a first direction away from straight brace or generally horizontal divider (2010) and a second set of braces or dividers (3010) is positioned in a second direction away from straight brace or generally horizontal divider (2010). Although not shown in FIG. 1, other preferred embodiments of cage (2000) can have a single brace (3010) extending in a first direction away from straight brace (2010) and a single brace (3010) extending in a second direction away from straight brace (2010). Each brace (3010) has inward side (3012), first lateral side (3014), second lateral side (3016) and outward side (3018). Intersection of first converging sides (3014) and inward sides (3012) create first corners (3020) and intersection of inward sides (3012) and second converging sides (3016) create second corners (3022), such that each brace (3010) has first corner (3020) and second corner (3022). First universal corner post (2030) contacts first corners (2020 and 3020) and second universal corner post (2040) contacts second corners (2022 and 3022). In the embodiment disclosed in FIG. 1, first universal corner post (2030) is angled at about ninety degrees to simultaneously connect with inward side (2012) and first lateral side (2014) of generally horizontal divider (2010) and inward sides (3012) and first lateral sides (3014) of dividers (3010). Second universal corner post (2040) is angled at about ninety degrees to simultaneously connect with inward side (2012) and second lateral side (2016) of straight brace (2010) and inward sides (3012) and second lateral sides (3016) of braces (3010).

In select embodiments, one or more first side universal posts (2050) contact first lateral sides (2014 and 3014) of cage (2000), one or more second side universal posts (2070) contact second lateral sides (2016 and 3016) of cage (2000) and one or more inward universal posts (2072) contact inward sides (2012 and 3012) of cage (2000). One of the first side universal posts (2050) is generally proximate outward side (2018) of brace (2010) and outward sides (3018) of braces (3010). In a similar vein, one of the second side universal posts (2070) is generally proximate outward side (2018) of brace (2010) and outward sides (3018) of braces (3010).

Inward support (2200) of cage (2000) includes inward side (2012) of straight brace (2010), inward sides (3012) of braces (3010) and universal corner posts (2030 and 2040). First lateral support (2240) of cage (2000) includes lateral side (2014) of straight brace (2010), lateral sides (3014) of braces (3010), universal corner post (2030) and one or more universal posts (2050). Second lateral support (2260) of cage (2000) includes lateral side (2016) of straight brace (2010), lateral sides (3016) of braces (3010), universal corner post (2040) and one or more universal posts (2070). Outward support (2220) of cage (2000) includes outward side (2018) of straight brace (2010), outward sides (3018) of braces (3010), an outward universal post (2050) and an outward universal post (2070).

Select preferred embodiments of cage (2000) can also include inward universal posts (2072) contacting generally inward side (2012) of generally horizontal divider (2010) and inward sides (3012) of dividers (3010). Still other preferred embodiments of cage (2000) can include one or more ties (2074) contacting universal corner posts (2030, 2040) and universal posts (2050, 2070 and 2072). Most preferably, ties (2074) are positioned on the inward sides of the universal posts (2030, 2040, 2050, 2070 and 2072).

The combination of straight brace (2010), braces (3010), universal corner posts (2030 and 2040), universal posts (2050, 2070 and 2072) and ties (2074) creates openings of more than one cross-sectional area about the outer border of cage (2000) - allowing the surgeon to see through the openings, prior to the addition of osteogenic or other substances into implant (2000).

In the preferred embodiment exemplified in FIG. 1, cage (2000) is provided with a plurality of brakes (2080) integral with one or more outward sides (2018 and 3018) of dividers (2010 and 3010). However, in other preferred embodiments of current invention, brakes (2080) need not be integral with outward sides (2018 and 3018) of braces (2010 and 3010). In other words, brakes (2080) can be affixed with outward universal posts (2050 and/or 2070) in any manner acceptable in the art.

As shown in FIG. 1, one or more brakes (2080) are provided with bore (2090). In the practice of the present invention, one or more brakes (2080) can extend laterally beyond first lateral support (2240) or one or more brakes (2080) can extend laterally beyond second lateral support (2260) or one or more brakes (2080) can extend laterally beyond first lateral support (2240) and second lateral support (2260). Brakes (2080) assist the surgeon in minimizing potential damage to the spinal cord from over-insertion of the implant into the surgically created cavity.

Depending on predetermined engineering parameters, as shown in FIG. 2, brake (2080) can include bore (2090) that is perpendicular to outward side (2082) and inward side (2084) of brake (2080) or brake (2080) can include bore (2090) that is angled at other than perpendicular from the outward side (2082) and inward side (2084) of brake (2080). It has been discovered that bore (2090) can be angled from about 1 degree to about 60 degrees away from the bore's reference axis (2086-2086), where the reference axis (2086-2086) equates to the perpendicular axis between outward side (2082) and inward side (2084) of brake (2080).

In select preferred embodiments, bores (2090) of brakes (2080) associated with first set of braces or superior dividers (3010) are angled upward from outward sides (2082) through inward sides (2084) of brakes (2080) and bores (2090) of brakes (2080) associated with second set of braces or inferior dividers (3010) are angled downward from outward sides (2082) through inward sides (2084) of brakes (2080). Bores (2090) function to receive a fastener, such as a screw (not shown in this view), that assists in securing the implant to bone.

Returning to FIG. 1, preferred embodiments of the current invention can be provided with a bar (2100) proximate to and parallel with outward side (2018) of straight brace (2010) and one or more bars (2100) proximate to and parallel with one or more outward sides (3018) of braces (3010). Bars (2100) contact first side universal post (2050) proximate outward side (2018) of brace (2010) and outward sides (3018) of braces (3010) and second side universal post (2070) proximate outward sides (2018) of brace (2010) and outward sides (3018) of braces (3010). Extensions (2102) of bars (2100) extend laterally beyond first lateral support (2240) or one or more extensions (2102) can extend laterally beyond second lateral support (2260) or one or more extensions (2102) can extend laterally beyond first lateral support (2240) and second lateral support (2260). Among other things, it has been discovered that the combination of outward sides' brakes (2080) and extensions (2102) can function as one or more grips for cage (2000). The grips enhance engagement of surgical instruments with implant (2000). Although not shown in FIG. 1, extensions (2102) can also be provided with one or more bores with structures similar to those previously enabled for bores (2090). It has also been discovered that the combination of outward sides (2018 and 3018) and bars (2100) enhances the cage's resistance to bending, twisting or torsion.

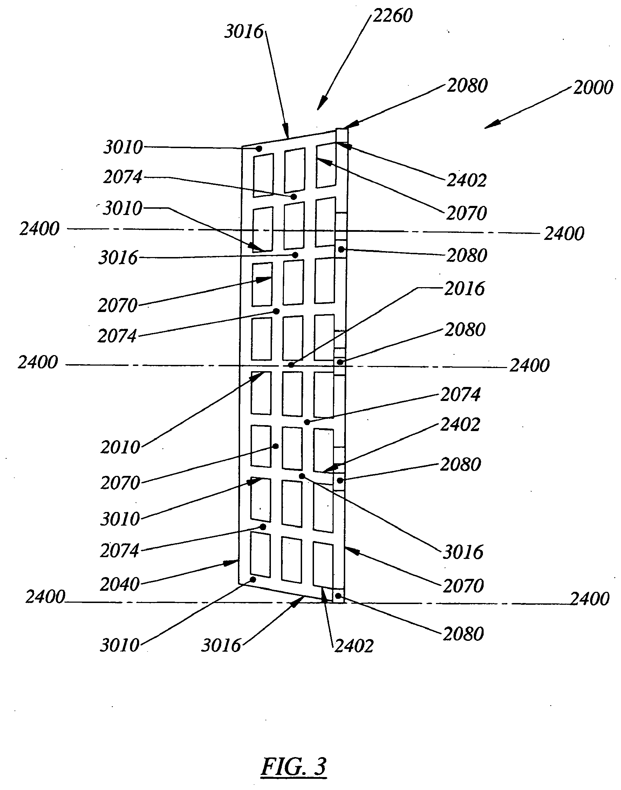

FIG. 3 is a lateral view of a preferred embodiment of second lateral support (2260) of implant (2000). Although not shown in this view, for this embodiment, first lateral support (2240) is identical to second lateral support (2260). As shown, second lateral support (2260) includes universal corner post (2040), universal posts (2070), brakes (2080), second lateral side (2016) of straight brace (2010), second lateral sides (3016) of first set of angled braces (3010), second lateral sides (3016) of second set of angled braces (3010) and ties (2074). Axis lines 2400-2400 represent horizontal. Inward sides of angled braces (3010) are closer to straight brace (2010) than outward sides of angled braces (3010). Each angled brace (3010) of the first set of angled braces (3010) diverges toward the straight brace or generally horizontal divider (2010) at angles (2402) from about 1 degree to about 10 degrees from horizontal. Each angled brace (3010) of the second set of angled braces (3010) diverges toward the straight brace or generally horizontal divider (2010) at angles (2402) from about 1 degree to about 10 degrees from horizontal. Other preferred embodiments can include ties (not shown in FIG. 3) that contact universal corner post (2040) and universal posts (2070) as well other universal corner posts and universal posts (not shown in this view).

Wedge-like structures of preferred embodiments increase contact surface area between the implant and tissue surrounding the surgically created cavity. The increased contact area enhances spinal stabilization while the increased rigidity improves bony fusion. Further, testing reveals that the cage's wedge-like structure is capable of withstanding greater compressive loads than round or oval implants.

Turning to the preferred embodiment disclosed in FIG. 4, implant (2000) is provided with a superior plate (1100) attached to outward side (3018) of superior brace (3010). In similar fashion, inferior plate (1200) is attached to outward side (3018) of inferior brace (3010). Upper plate (1100) has two bores (1102) for receiving fasteners, such as screws, to assist in securing cage (2000) to bone. Lower plate (1200) includes two bores (1202) for receiving fasteners that assist in securing cage (2000) to bone. Although upper plate (1100) and lower plate (1200) are shown with a plurality of bores, Applicant's current invention can function when upper plate (1100) and lower plate (1200) each include only a single bore (1102, 1202).

As shown in the embodiment of FIG. 4, superior plate (1100) extends upward from superior brace or divider (3010) and inferior plate (1200) depends downward from inferior brace or divider (3010). In select embodiments, superior plate (1100) extends upward in a plane perpendicular to outward side (3018) of superior brace (3010) and inferior plate (1200) depends downward in a plane perpendicular to outward side (3018) of inferior divider (3010). Although not shown FIG. 4, in other embodiments, plate (1100) can be extended 2-3 millimeters outward from superior brace (3010) before plate (1100) extends upward from superior brace (3010). In a similar manner, inferior plate (1200) can be extended 2-3 millimeters outward from inferior brace (3010) before plate (1200) depends downward from inferior brace (3010). When the need arises, outward extension of superior plate (1100) and inferior plate (1200) allows cage (2000) to securely fit within a deeper surgical cavity. Straight brace (2010) and intermediate superior and intermediate inferior braces (3010) include one or more brakes (2080) having bore (2090). As shown, brakes (2080) extend laterally beyond first lateral support (2240) and second lateral support (2260) of implant (2000).

With reference to FIG. 5, plate (1100) can include one or more bores (1102) where each bore (1102) is perpendicular to outward side (1104) and inward side (1106) of plate (1100) or plate (1100) can include one or more bores (1102) that are angled at other than perpendicular from the outward side (1104) and inward side (1106) of plate (1100). By way of example, bores (1102) can be angled from about 1 degree to about 60 degrees away from the bore's reference axis (1110), where the reference axis (1110-1110) equates to the perpendicular axis between outward side (1104) and inward side (1106) of plate (1000). Plate (1200) and one or more bores (1202) are manufactured in a similar fashion to plate (1100) and one or more bores (1102).

In select preferred embodiments, bores (1102) of plate (1100) are angled upward from outward side (1104) through inward side (1106) of plate (1100) and bores (1202) of plate (1200) are angled downward from the outward side of plate (1200) through the inward side of plate (1200) One or more bores (1102, 1202) function to receive a fastener, such as a screw (not shown in this view) that assists in securing the implant to bone. Select preferred embodiments can include brakes (2080) that are not integral with outward sides (2018 and 3018) of braces (2010 and 3010).

With reference to the preferred embodiment portrayed in FIG. 6, implant (2000) is provided with superior brace (3010) that includes a plurality of bores (1092) integral with outward side (3018) and inward edge (3018A) of superior brace (3010). Inferior brace (3010) includes a plurality of bores (1092) integral with outward side (3018) and inward edge (3018A) of inferior divider (3010). Bores (1092) are inset from first lateral support (2240) and second lateral support (2260) of cage (2000). As depicted in FIG. 7, superior brace (3010) includes one or more bores (1092) where each bore (1092) is angled at other than perpendicular from the outward side (3018) and inward edge (3018A) of brace (3010). Bores (1092) can be angled from about 1 degree to about 60 degrees away from the bore's reference axis (1096-1096), where the reference axis (1096-1096) equates to the perpendicular axis between outward side (3018) and inward edge (3018A) of brace (3010). Inferior brace (3010) and one or more bores (1092) are manufactured in a similar fashion to superior brace (3010) and one or more bores (1092). In the preferred embodiments, bores (1092) of superior brace (3010) are angled upward from outward side (3018) through inward edge (3018A) of superior brace (3010) and bores (1092) of inferior brace (3010) are angled downward from the outward side (3018) through inward edge (3018A) of inferior brace (3010). Bores (1092) function to receive a fastener, such as a screw (not shown in this view) that assists in securing the implant to bone.

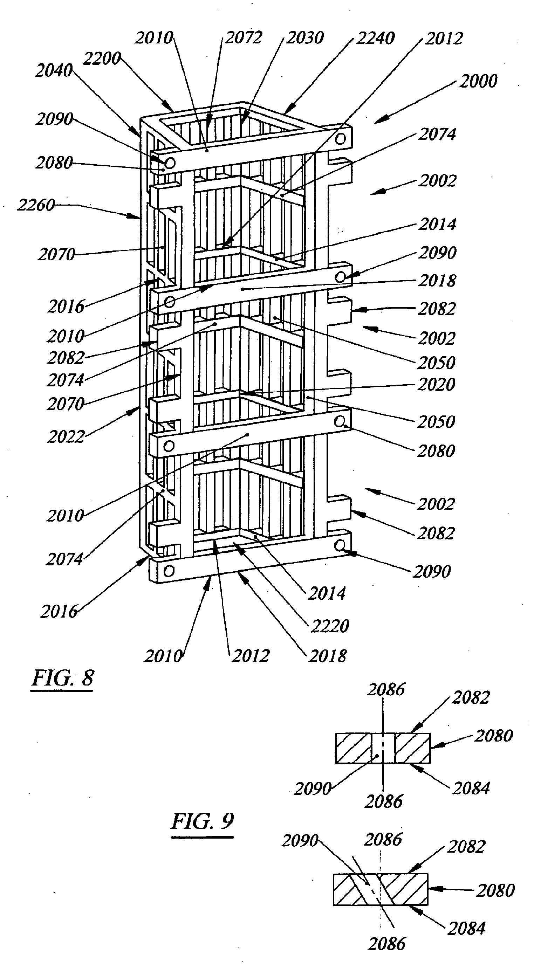

Among other things, FIG. 8 enables a cage (2000) having a series of trapezoidal dividers (2010) and a plurality of receptacles (2002) that can be packed with osteogenic materials or other substances prior to the completion of spinal surgery. Generally horizontal dividers or braces (2010) have inward sides (2012) (after insertion into the surgically created cavity, positioned near the dura mater of the spinal cord), outward sides (2018) (proximate the surgeon after insertion into the surgically created cavity), first converging or lateral sides (2014) and second converging or lateral sides (2016). Intersection of first lateral sides (2014) and inward sides (2012) create first corners (2020) and intersection of inward sides (2012) and second lateral sides (2016) creates second corners (2022), such that each brace (2010) has first corner (2020) and second corner (2022).

First universal corner post (2030) contacts first corners (2020) and second universal corner post (2040) contacts second corners (2022). As shown in FIG. 8, first universal corner post (2030) is angled at about ninety degrees to simultaneously connect with inward sides (2012) and first lateral sides (2014) of generally horizontal dividers (2010) and second universal corner post (2040) is angled at about ninety degrees to simultaneously connect with inward sides (2012) and second lateral sides (2016) of braces (2010). In other preferred embodiments, universal corner posts (2030 and 2040) are not angled at about ninety degrees.

In select embodiments, one or more first side universal posts (2050) contact first lateral sides (2014) of cage (2000), one or more second side universal posts (2070) contact second lateral sides (2016) of cage (2000) and one or more inward universal posts (2072) contact inward sides (2012) of cage (2000). One of the first side universal posts (2050) is generally proximate outward sides (2018) of brace (2010), and in a similar vein, one of the second side universal posts (2070) is generally proximate outward sides (2018) of braces (2010).

Inward support (2200) of cage (2000) includes inward sides (2012) of braces (2010) and universal corner posts (2030 and 2040). First lateral support (2240) of cage (2000) includes lateral sides (2014) of braces (2010), universal corner post (2030) and one or more universal posts (2050). Second lateral support (2260) of cage (2000) includes lateral sides (2016) of braces (2010), universal corner post (2040) and one or more universal posts (2070). Outward support (2220) of cage (2000) includes outward sides (2018) of braces (2010), an outward universal post (2050) and an outward universal post (2070).

Select preferred embodiments of cage (2000) can also include inward universal posts (2072) contacting generally inward sides (2012) of generally horizontal dividers (2010). Still other preferred embodiments of cage (2000) can include one or more ties (2074) contacting universal corner posts (2030, 2040) and universal posts (2050, 2070 and 2072). Most preferably, ties (2074) are positioned on the inward sides of the universal posts (2030, 2040, 2050, 2070 and 2072).

The combination of braces (2010), universal corner posts (2030 and 2040), universal posts (2050, 2070 and 2072) and ties (2074) creates openings of more than one cross-sectional area about the outer border of cage (2000) - allowing the surgeon to see through the openings, prior to the addition of osteogenic or other substances into implant (2000).

In the preferred embodiment exemplified in FIG. 8, cage (2000) is provided with a plurality of lateral extensions or brakes (2080 and 2082). As shown brakes (2080) are integral with one or more outward sides (2018) of dividers (2010). However, in other preferred embodiments of current invention, lateral extensions (2080) need not be integral with outward sides (2018) of braces (2010). In other words, lateral extensions (2080 and 2082) can be affixed with outward universal posts (2050 and/or 2070) in any manner acceptable in the art. The combination of lateral extensions (2080 and 2082) can function as a grip for cage (2000). It has been discovered that the use of the grip improves a surgical instrument's gripping of implant (2000).

As shown in FIG. 8, one or more brakes (2080 and 2082) are provided with bore (2090). In the practice of the present invention, one or more lateral extensions (2080 and 2082) extend laterally beyond first lateral support (2240) or one or more lateral extensions (2080 and 2082) extend laterally beyond second lateral support (2260) or one or more lateral extensions (2080 and 2082) extend laterally beyond first lateral support (2240) and second lateral support (2260). Along with providing a grip, lateral extensions (2080 and 2082) assist the surgeon in minimizing potential damage to the spinal cord from over-insertion of the implant into the surgically created cavity.

Depending on predetermined engineering parameters, as shown in FIG. 9, lateral extension (2080) can include bore (2090) that is perpendicular to outward side (2082) and inward side (2084) of brake (2080) or lateral extension (2080) can include bore (2090) that is angled at other than perpendicular from the outward side (2082) and inward side (2084) of brake (2080). It has been discovered that bore (2090) can be angled from about 1 degree to about 60 degrees away from the bore's reference axis (2086-2086), where the reference axis (2086-2086) equates to the perpendicular axis between outward side (2082) and inward side (2084) of lateral extension (2080). Although not shown in FIGS. 8 or 9, lateral extension (2082) can be provided with a bore having identical structures to those enabled for bore (2090).

In select preferred embodiments, bores (2090) of brakes (2080) associated with superior dividers (2010) can be angled upward from outward sides (2082) through inward sides (2084) of brakes (2080) and bores (2090) of brakes (2080) associated with inferior dividers (2010) can be angled downward from outward sides (2082) through inward sides (2084) of brakes (2080). Bores (2090) function to receive a fastener, such as a screw (not shown in this view), that assists in securing the implant to bone.

The preferred embodiment disclosed in FIG. 10 is similar to the preferred embodiment disclosed in FIG. 8. As shown in the FIG. 10 embodiment, bars (2085 are integral with lateral extensions (2082). In other embodiments, bars (2085) are not integral with lateral extensions (2082). As previously indicated, bores (2098) can be provided structures identical to those enabled for bore (2090).

As portrayed in FIG. 10, bars (2085) are proximate to and parallel with outward sides (2018) of braces (2010). Bars (2085) contact first side universal post (2050) proximate outward sides (2018) of brace (2010) and second side universal post (2070) proximate outward sides (2018) of brace (2010). Among other things, it has been discovered that the combination of outward sides (2018) and bars (2085) enhances the cage's resistance to bending, twisting or torsion. Further, testing reveals that the cage's structure is capable of withstanding greater compressive loads than round or oval implants.

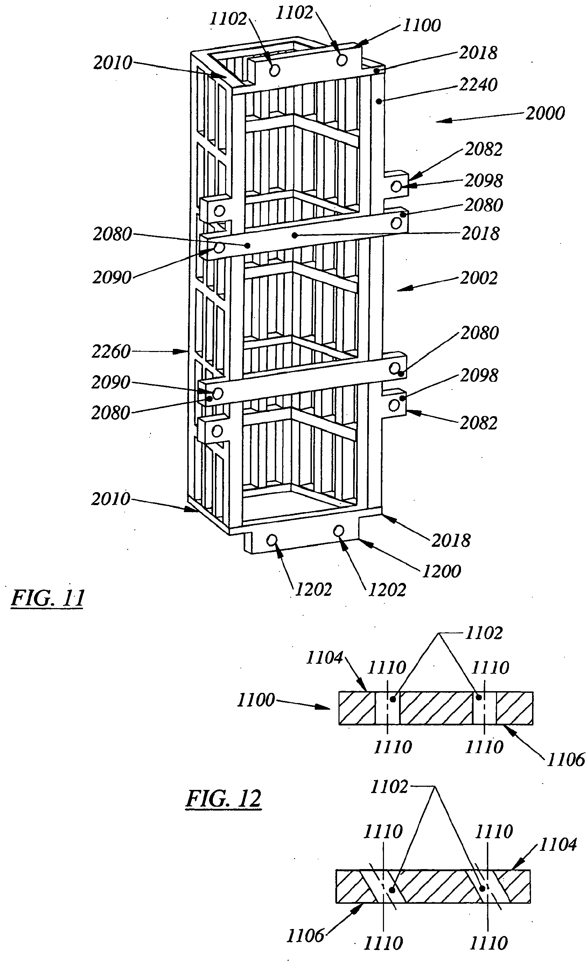

Turning to the preferred embodiment disclosed in FIG. 11, implant (2000) is provided with a superior plate (1100) attached to outward side (2018) of superior brace (2010). In similar fashion, inferior plate (1200) is attached to outward side (2018) of inferior brace (2010). Upper plate (1100) has two bores (1102) for receiving fasteners, such as screws, to assist in securing cage (2000) to bone. Lower plate (1200) includes two bores (1202) for receiving fasteners that assist in securing cage (2000) to bone. Although upper plate (1100) and lower plate (1200) are shown with a plurality of bores, Applicant's current invention can function when upper plate (1100) and lower plate (1200) each include only a single bore (1102, 1202). As shown, lateral extensions (2080 and 2082) extend laterally beyond first lateral support (2240) and second lateral support (2260) of implant (2000).

As shown in the embodiment of FIG. 11, superior plate (1100) extends upward from superior brace or divider (2010) and inferior plate (1200) depends downward from inferior brace or divider (2010). In select embodiments, superior plate (1100) extends upward in a plane perpendicular to outward side (2018) of superior brace (2010) and inferior plate (1200) depends downward in a plane perpendicular to outward side (2018) of inferior divider (2010). Although not shown FIG. 11, in other embodiments, plate (1100) can be extended 2-3 millimeters outward from superior brace (2010) before plate (1100) extends upward from superior brace (2010). In a similar manner, inferior plate (1200) can be extended 2-3 millimeters outward from inferior brace (2010) before plate (1200) depends downward from inferior brace (2010). Proximate intermediate braces (2010) are one or more lateral extensions (2080 and 2082) that can be provided with bores (2090 and 2098).

With reference to FIG. 12, the descriptions of bores (1102 and 1202) are identical to the description of bores (1102 and 1202) associated with the enablement portrayed in FIG. 5.

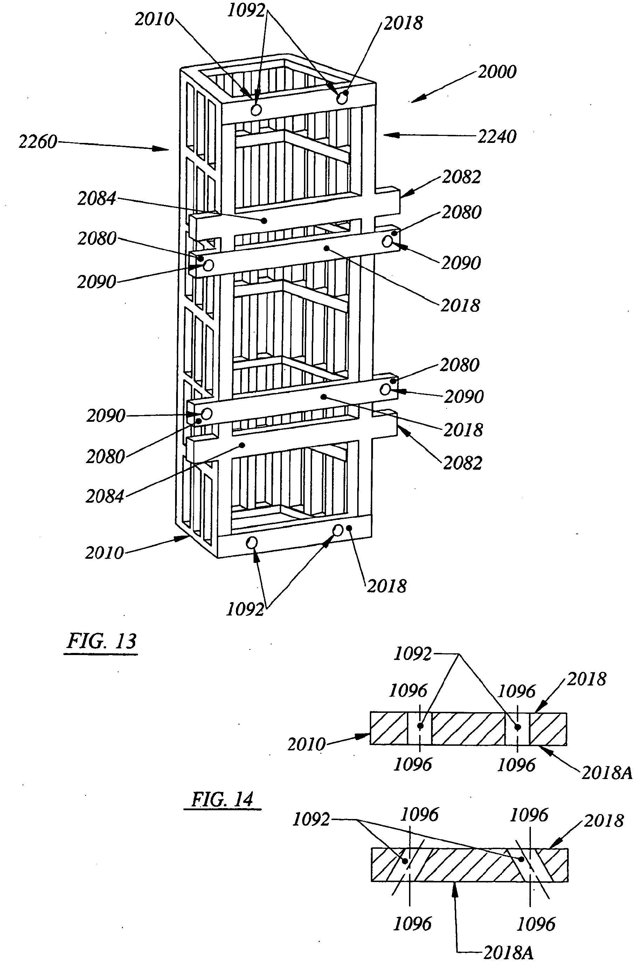

With reference to the preferred embodiment portrayed in FIG. 13, implant (2000) is provided with superior brace (2010) that includes a plurality of bores (1092) integral with outward side (2018) and inward edge (2018A) of superior brace (2010). Inferior brace (2010) includes a plurality of bores (1092) integral with outward side (2018) and inward edge (2018A) of inferior divider (2010). Bores (1092) are inset from first lateral support (2240) and second lateral support (2260) of cage (2000). As depicted in FIG. 14, superior brace (2010) includes one or more bores (1092) where each bore (1092) is angled at other than perpendicular from the outward side (2018) and inward edge (2018A) of brace (2010). Bores (1092) can be angled from about 1 degree to about 60 degrees away from the bore's reference axis (1096-1096), where the reference axis (1096-1096) equates to the perpendicular axis between outward side (2018) and inward edge (2018A) of brace (2010). In select preferred embodiments bores are coaxial with reference axis. Inferior brace (2010) and one or more bores (1092) are manufactured in a similar fashion to superior brace (2010) and one or more bores (1092). In the preferred embodiments, bores (1092) of superior brace (2010) are angled upward from outward side (2018) through inward edge (2018A) of superior brace (2010) and bores (1092) of inferior brace (2010) are angled downward from the outward side (2018) through inward edge (2018A) of inferior brace (2010). Bores (1092) function to receive a fastener, such as a screw (not shown in this view) that assists in securing the implant to bone.

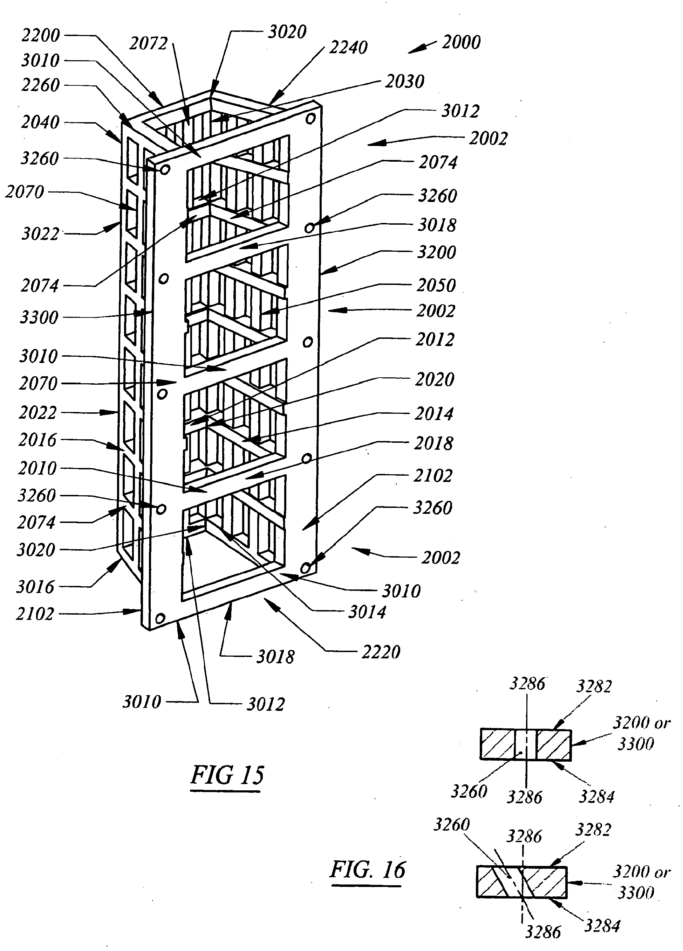

Among other things, FIG. 15 enables a cage (2000) having a series of trapezoidal dividers or braces (2010) and (3010) and a plurality of receptacles (2002) that can be packed. Generally horizontal divider or brace (2010) has inward side (2012) (after insertion into the surgically created cavity, positioned near the dura mater of the spinal cord), outward side (2018) (proximate the surgeon after insertion into the surgically created cavity), first converging or lateral side (2014) and second converging or lateral side (2016). Intersection of first lateral side (2014) and inward side (2012) creates first corner (2020) and intersection of inward side (2012) and second lateral side (2016) creates second corner (2022), such that straight brace (2010) has first corner (2020) and second corner (2022).

A first set of braces or dividers (3010) is positioned in a first direction away from straight brace or generally horizontal divider (2010) and a second set of braces or dividers (3010) is positioned in a second direction away from straight brace or generally horizontal divider (2010). Although not shown in FIG. 15, other preferred embodiments of cage (2000) can have a single brace (3010) extending in a first direction away from straight brace (2010) and a single brace (3010) extending in a second direction away from straight brace (2010). With respect to FIG. 15, the enablement of braces (2010 and 3010) and ties (2074) is identical to the description set forth regarding braces (2010 and 3010) and ties (2074) in FIG. 1.

The combination of straight brace (2010), braces (3010), universal corner posts (2030 and 2040), universal posts (2050, 2070 and 2072) and ties (2074) creates openings of more than one cross-sectional area about the outer border of cage (2000) - allowing the surgeon to see through the openings, prior to the addition of osteogenic or other substances into implant (2000).

In the preferred embodiment exemplified in FIG. 15, cage (2000) is provided with first lateral brake (3200) and second lateral brake (3300). Lateral brakes (3200 and 3300) assist the surgeon in minimizing potential damage to the spinal cord from over-insertion of implant (2000) into a surgically created cavity. First lateral brake (3200) extends laterally beyond first lateral sides (2014 and 3014) of braces (2010 and 3010) and second lateral brake (3300) extends laterally beyond second lateral sides (2016 and 3016) of braces (2010 and 3010) of implant (2000). In select preferred embodiments, lateral brakes (3200 and 3300) can also form part of outward support (2220) of cage (2000). Through experimentation and testing, it has been discovered that an optimal functionality of the present invention can be achieved when lateral brakes (3200 and 3300) are extended laterally from about one millimeter to about six millimeters beyond the lateral sides of cage (2000).

In select preferred embodiments, lateral brake (3200) is integral with outward universal post (2050) and lateral brake (3300) is integral with outward universal post (2070). As shown in FIG. 15, first lateral brake (3200) runs generally vertically from about superior brace or superior member (3010) of the series of trapezoidal braces (2010 and 3010) to about inferior brace or inferior member (3010) of the series of trapezoidal braces (2010 and 3010). Second lateral brake (3300) runs from about superior member (3010) of the series of trapezoidal braces (2010 and 3010) to about inferior member (3010) of the series of trapezoidal braces (2010 and 3010).

As shown in FIG. 15, lateral brakes (3200 and 3300) are provided with one or more bores (3260). Depending on predetermined engineering parameters and as shown in FIG. 16, first lateral brake (3200) and/or second lateral brake (3300) can include one or more bores (3260) that are perpendicular to outward sides (3282) and inward sides (3284) of lateral brakes (3200 and 3300). In select preferred embodiments, lateral brakes (3200 and 3300) can include one or more bores (3260) that are angled at other than perpendicular from the outward sides (3282) and inward sides (3284) of lateral brakes (3200 and 3300). It has been discovered that bores (3260) can be angled from about 1 degree to about 60 degrees away from the bore's reference axis (3286-3286), where the reference axis (3286-3286) equates to the perpendicular axis between outward sides (3282) and inward sides (3284) of lateral brakes (3200 and 3300). In select preferred embodiments, bores (3260) of lateral brakes (3200 and 3300) proximate superior member (3010) of the series of trapezoidal braces (2010 and 3010) are angled upward and bores (3260) proximate inferior member (3010) of the series of trapezoidal braces (2010 and 3010) are angled downward. Bores (3260) function to receive a fastener, such as a screw (not shown in this view), that assists in securing the implant to bone.

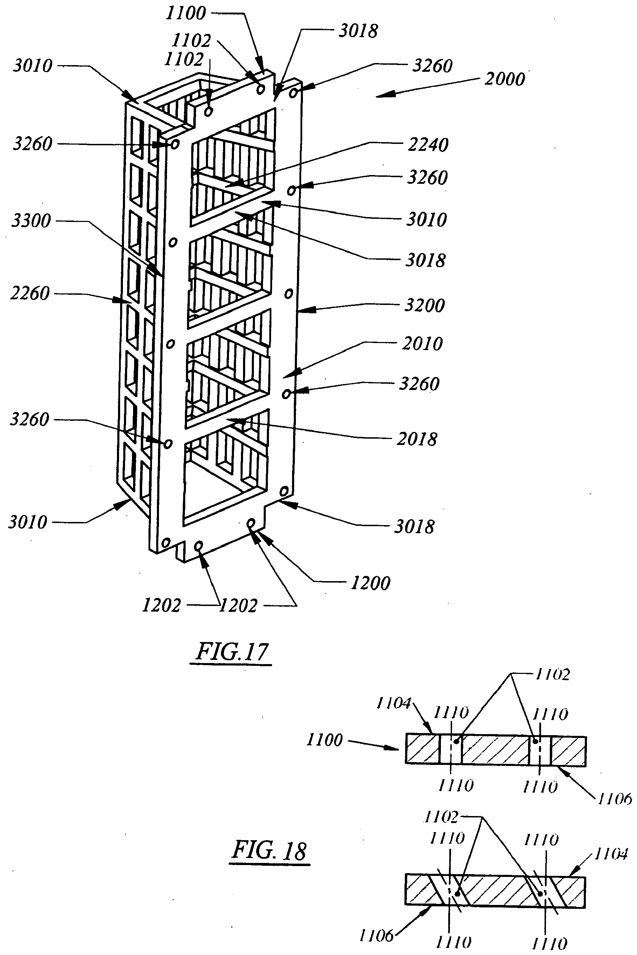

The preferred embodiment disclosed in FIG. 17 that is similar to the preferred embodiment disclosed in FIG. 15. The FIG. 17 embodiment portrays cage (2000) having first lateral brake (3200) and second lateral brake (3300). First lateral brake (3200) extends laterally beyond first lateral support (2240) of cage (2000) and second lateral brake (3300) extends laterally beyond lateral support (2260) of cage (2000). First lateral brake (3200) and second lateral brake (3300) are provided with one or more bores (3260).

As shown in FIG. 17, implant (2000) is also provided with a superior plate (1100) extending upward from superior member (3010) of the series of trapezoidal braces (2010 and 3010). In similar fashion, inferior plate (1200) depends downwardly from inferior member (3010) of the series of trapezoidal braces (2010 and 3010). Upper plate (1100) has two bores (1102) for receiving fasteners, such as screws, to assist in securing cage (2000) to bone. Lower plate (1200) includes two bores (1202) for receiving fasteners that assist in securing cage (2000) to bone. Although upper plate (1100) and lower plate (1200) are shown with a plurality of bores, Applicant's current invention can function when upper plate (1100) and lower plate (1200) each include only a single bore (1102, 1202) or no bores.

With reference to FIG. 18, the descriptions of bores (1102 and 1202) are identical to the description of bores (1102 and 1202) associated with the enablement portrayed in FIGS. 5 and 12.

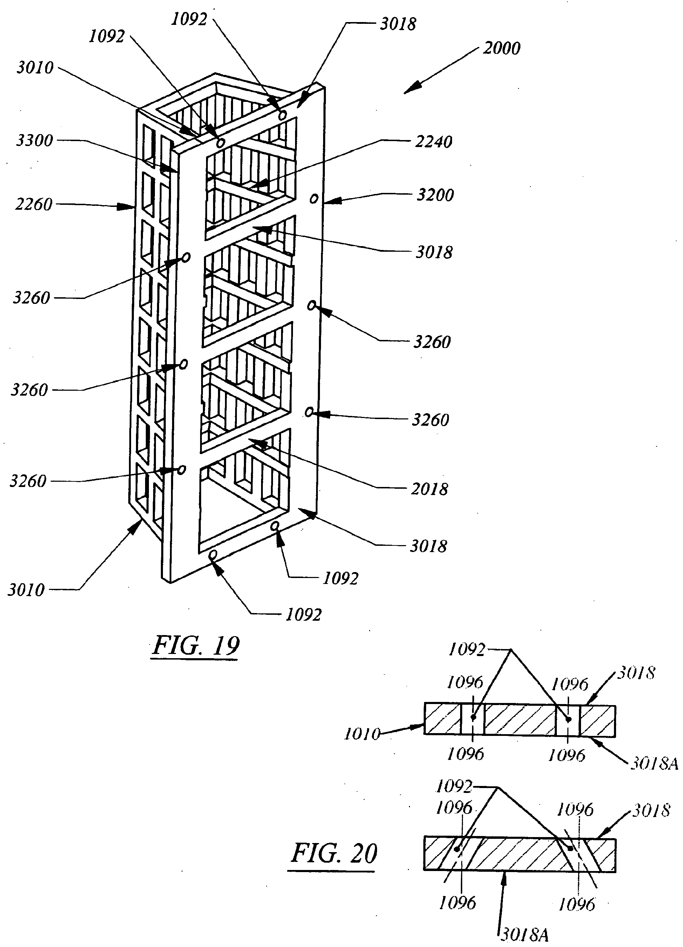

The preferred embodiment disclosed in FIG. 19 is similar to the preferred embodiment disclosed in FIGS. 15 and 17. The FIG. 19 embodiment portrays cage (2000) including first lateral brake (3200) and second lateral brake (3300). First lateral brake (3200) extends laterally beyond first lateral support (2240) of cage (2000) and second lateral brake (3300) extends laterally beyond lateral support (2260) of cage (2000). First lateral brake (3200) and second lateral brake (3300) are provided with one or more bores (3260).

With reference to the preferred embodiments portrayed in FIGS. 19 and 20, implant (2000) is provided with superior member (3010) of the series of trapezoidal braces (2010 and 3010) that includes a plurality of bores (1092) integral with outward side (3018) and inward edge (3018A) of superior member (3010). Inferior member (3010) of the series of trapezoidal braces (2010 and 3010) includes a plurality of bores (1092) integral with outward side (3018) and inward edge (3018A) of inferior member (3010). Bores (1092) are inset from first lateral support (2240) and second lateral support (2260) of cage (2000).

With reference to FIG. 20, the descriptions of bores (1092) for superior and inferior braces (3010) are identical to the description of bores (1092) associated with the enablement portrayed in FIG 14.

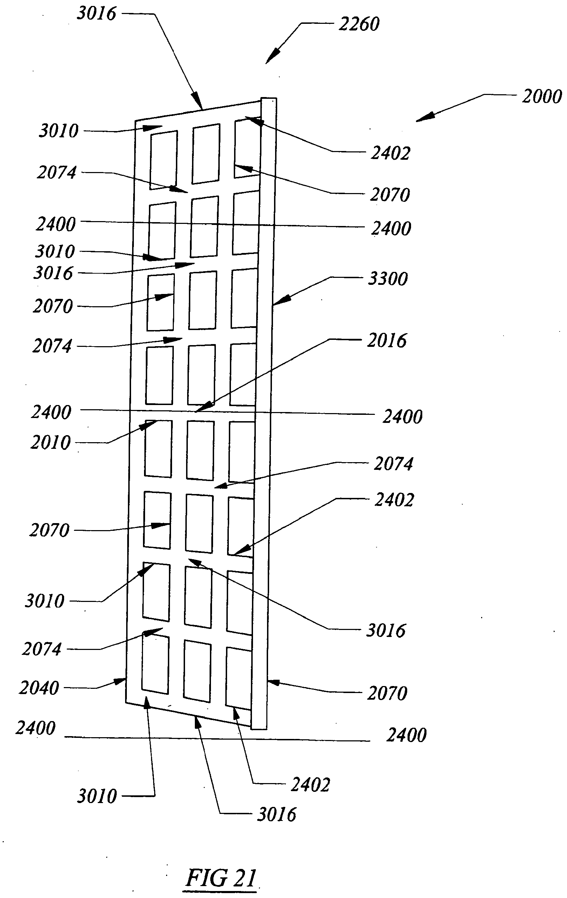

FIG. 21 is a lateral view of an embodiment of second lateral support (2260) of a preferred embodiment of implant (2000). Although not shown in this view, for this preferred embodiment, first lateral support (2240) is identical to second lateral support (2260). As shown, second lateral support (2260) includes universal corner post (2040), universal posts (2070), second lateral side (2016) of straight brace (2010), second lateral sides (3016) of first set of angled braces (3010), second lateral sides (3016) of second set of angled braces (3010) and ties (2074). Second lateral brake (3300) is also portrayed.

With respect to FIG. 21, axis lines 2400-2400 represent horizontal. Inward sides of angled braces (3010) are closer to straight brace (2010) than outward sides of angled braces (3010). Each angled brace (3010) of the first set of angled braces (3010) diverges toward the straight brace or generally horizontal divider (2010) at angles (2402) from about 1 degree to about 10 degrees from horizontal. Each angled brace (3010) of the second set of angled braces (3010) diverges toward the straight brace or generally horizontal divider (2010) at angles (2402) from about 1 degree to about 10 degrees from horizontal.

Within the scope of the present invention, preferred embodiments enabled in FIGS. 1, 4, 6, 15, 17, 19, 22, 29 and 30 can be manufactured such that: 1) the first set of braces (3010) and the second set of braces are generally horizontal; 2) the first set of braces (3010) or the second set of braces (3010) can diverge toward the centralized brace (2010) while the non-diverging set of braces (3010) remains generally horizontal; or 3) the first set of braces (3010) and the second set of braces (3010) diverge toward the centralized brace (2010).

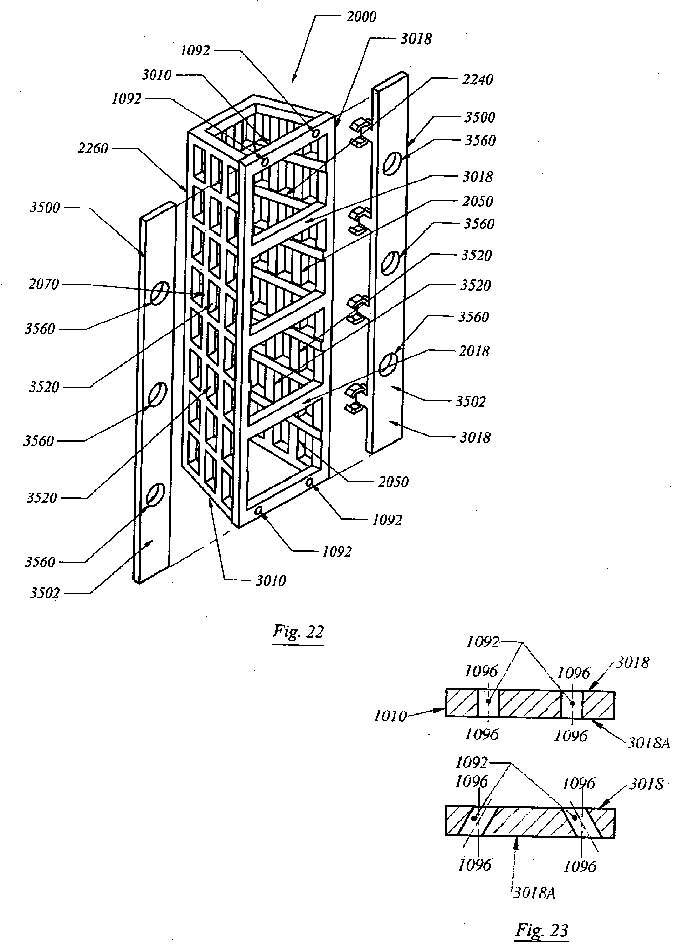

In many respects, the preferred embodiment disclosed in FIG. 22 is similar to the preferred embodiment disclosed in FIGS 15, 17 and 19. FIG. 22 is an exploded view of cage (2000) that utilizes a pair of connectable lateral brakes (3500). When connected to cage (2000), connectable lateral brakes (3500) extend laterally beyond lateral supports (2240 and 2260), respectively, of cage (2000). Preferred embodiments of connectable lateral brakes (3500) are provided with one or more bores (3560). Among other things, FIG. 22 portrays outward sides (3502) of connectable lateral brakes (3500).

With reference to the preferred embodiments portrayed in FIGS. 22 and 23, implant (2000) is provided with superior member (3010) of the series of trapezoidal braces (2010 and 3010) that includes a plurality of bores (1092) integral with outward side (3018) and inward edge (3018A) of superior member (3010). Inferior member (3010) of the series of trapezoidal braces (2010 and 3010) includes a plurality of bores (1092) integral with outward side (3018) and inward edge (3018A) of inferior member (3010). Bores (1092) are inset from first lateral support (2240) and second lateral support (2260) of cage (2000).

With reference to FIG. 23, the descriptions of bores (1092) for superior and inferior braces (3010) are identical to the description of bores (1092) associated with the enablement portrayed in FIGS 14 and 20.

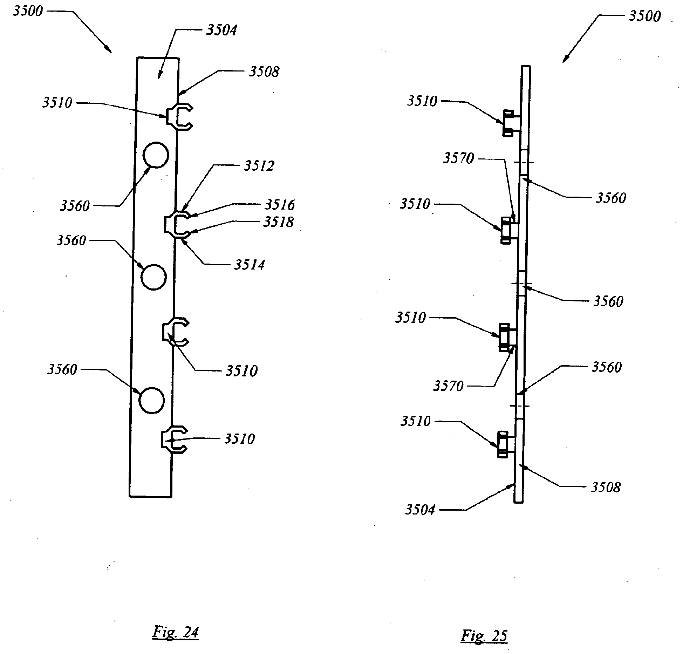

FIG. 24 is a perspective of inward side (3504) of a connectable lateral brake (3500). Attached to inward side (3504) of connectable lateral brake (3500) are connectors (3510) for connecting connectable lateral brakes (3500) to first lateral support (2240) and/or second lateral support (2260) of cage (2000). Although other means can be used to secure connectable lateral brakes (3500) to implant (2000), in FIG. 24, clips (3510) are enabled. Clip (3510) includes first projection (3512) and second projection (3514) that extend beyond edge (3508) of connectable lateral brake (3500). Tips (3516 and 3518) of clips (3510) are biased toward each other such that when clips (3510) are forced about either a universal post (2050) of first lateral support (2040) and/or a universal post (2070) of second lateral support (2260), connectable lateral brakes (3500) are secured to cage (2000). In select preferred embodiments projections, (3512 and 3514) of clips (3510) are engineered to have dimensions that virtually fill spaces (3520) proximate to universal posts (2050) and/or (2070) where clips (3510) have been attached. By way of illustration and not limitation, clips or connectors (3510) can have a height of about 2 millimeters, a depth of 4 millimeters and a width of about 2 millimeters.

FIG. 25 is a frontal view of edge (3508) of connectable lateral brake (3500). Arm (3570) is attached to inward side (3504) of connectable lateral brake (3500) and connector (3510). Welds or any other means acceptable in the art can attach arm (3570) to inward side (3504) and connector (3510). In select preferred embodiments, arm (3570) extends clip (3510) for a length from inward side (3504) such that clip (3510) is positioned for secure engagement with universal posts (2050) and/or (2070), other than the outermost universal posts (2050) or (2070), and spaces (3520) of first lateral support (2240) and/or second lateral support (2260) of cage (2000).

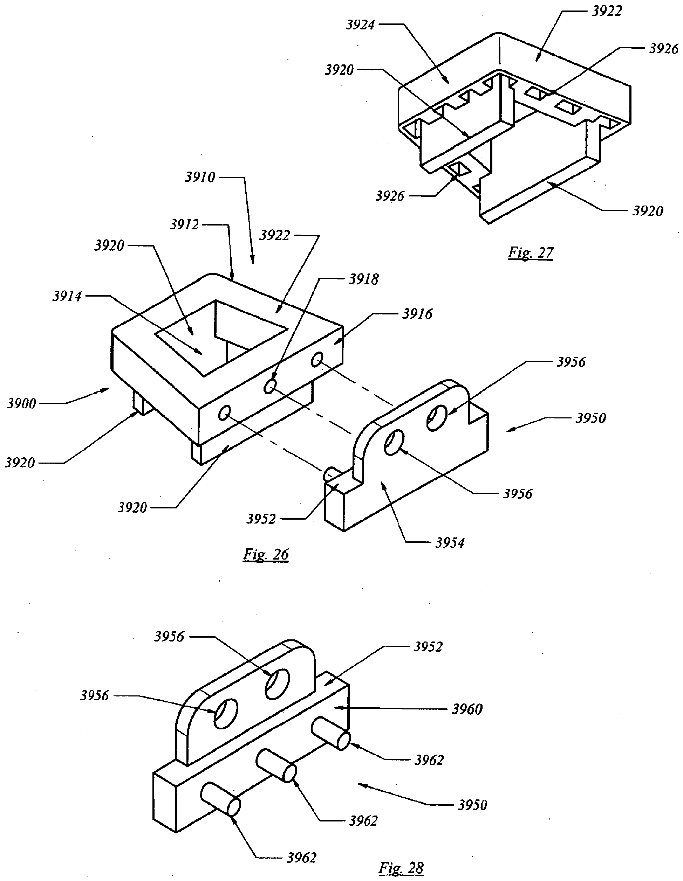

FIG. 26 is an exploded frontal perspective that enables and shows an end cap (3900). End cap (3900) can assist in securing the spinal implant to vertebra. As shown in FIG. 26, end cap (3900) comprises bridge (3910) and coupler (3950).

Bridge (3910) has outermost section (3912) that surrounds a generally trapezoidal aperture (3914). Surgeon facing side (3916) of outermost section (3912) is provided with a plurality of bores (3918). As shown in FIG. 26, surgeon facing side (3916) of bridge (3910) is provided with three bores (3918), but other embodiments of surgeon facing side (3916) can be provided with one or more bores (3918).

Preferred embodiments of bridge (3910) are provided with two or more docking slides (3920) that extend from outermost section (3912) of bridge (3910) toward a corresponding opening proximate a lengthwise end of the spinal implant (not shown in this view). Docking slides (3920) are engineered to securely fit within the corresponding opening proximate a lengthwise end of the spinal implant.

FIG. 27 is a perspective of end cap (3900) and implant proximate surface (3924) opposite implant distal surface (3922) of outermost section (3912) of bridge (3910). Select embodiments of implant proximate surface (3924) of bridge (3910) are provided with bores (3926). Bores (3926) can reciprocate with spikes of the spinal implant (not shown in this view).

Returning to FIG. 26, coupler (3950) includes spacer (3952) and frontal plate (3954). Frontal plate (3954) is provided with holes (3956) capable of receiving fasteners (not shown in this view).

FIG. 28 is a perspective view of bridge facing side of coupler (3950). Inward side (3960) of coupler (3950) is provided with three pins (3962) corresponding to bores (3918) of surgeon facing side (3916) of outermost section (3912) of bridge (3910). Pins (3962) are attached to inward side (3960) in any manner acceptable in the art. As shown in FIG. 28, coupler (3950) is provided with three pins (3962), but other embodiments of coupler (3950) can be provided with one or more pins (3962).

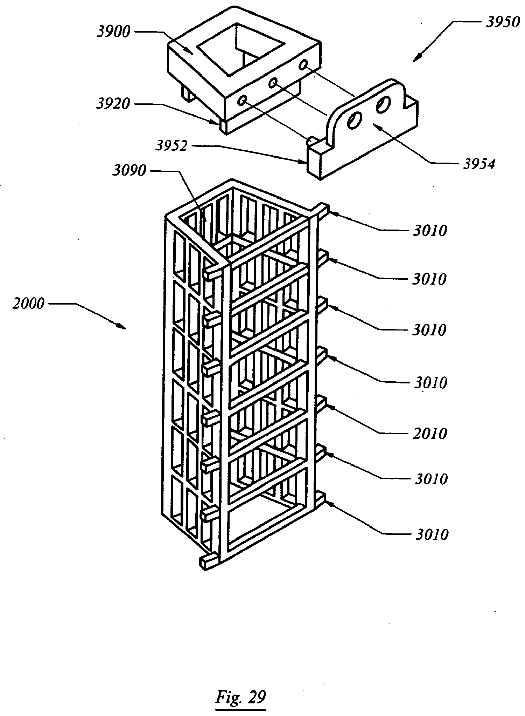

FIG. 29 is an exploded view of a preferred embodiment of an end cap (3900) and cage (2000). As shown, end cap (3900) is attached to superior brace (3010) of cage (2000). Docking slides (3920) securely fit within trapezoidal opening (3090) of cage (2000). Spacer (3952) of coupler (3950) causes frontal plate (3954) to be anterior to cage (2000). Fasteners (not shown in this view), such as screws, are inserted through holes (3956) to secure end cap (3900) to vertebra (not shown in this view). End cap (3900) is compatible with either inferior or superior trapezoidal openings (3090) of cage (2000).

Preferred embodiments of outermost section (3912) of bridge (3910) of end cap (3900) can have a height or thickness of from about 2 millimeters to about 5 millimeters. In select preferred embodiments, the thickness of outermost section (3912) is a consistent height. In other preferred embodiments, outermost section (3912) of bridge (3910) is provided with surgeon facing side (3932) of greater thickness than vertebra proximate side (3934). In some preferred embodiments, lateral sides of outermost section (3912) of bridge (3910) can taper between surgeon facing side (3932) and vertebra proximate side (3934).

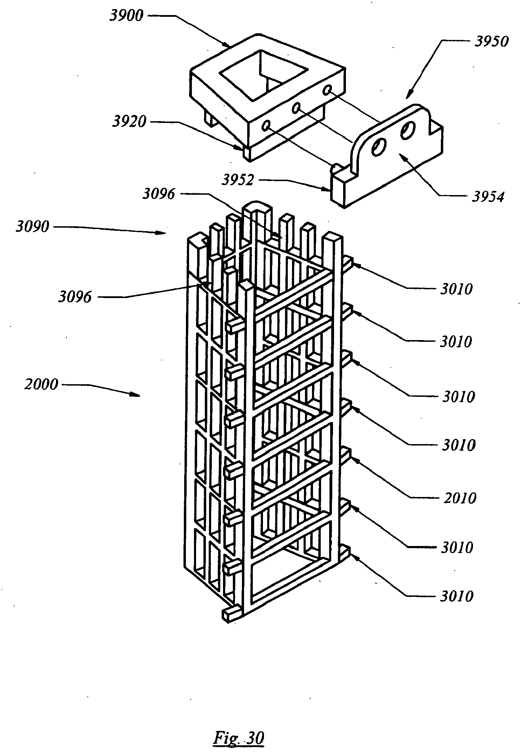

FIG. 30 is an exploded view of another preferred embodiment of an end cap (3900) and cage (2000). Spikes (3096) are created when cage (2000) is severed along a longitudinal section generally parallel brace (3010). Although not shown in this view, bores (3926) of bridge (3910) reciprocate with spikes (3096) as docking slides (3920) slide into trapezoidal opening (3090) to secure end cap (3900) to cage (2000). Spacer (3952) of coupler (3950) causes frontal plate (3954) to be anterior to cage (2000). Fasteners (not shown in this view) are inserted through holes (3956) to secure end cap (3900) to vertebra (not shown in this view). Embodiments of end cap (3900) are compatible with either inferior or superior trapezoidal openings (3090) of cage (2000).

The coupling of end cap (3900) and cage (2000) effectively increases the length of the spinal implant. In select preferred embodiments, the combination of end cap (3900) and cage (2000) can assist in creation of lordosis at one or more ends of the spinal implant. When connected to vertebra, end cap (3900) can also enhance stability of the spinal implant.

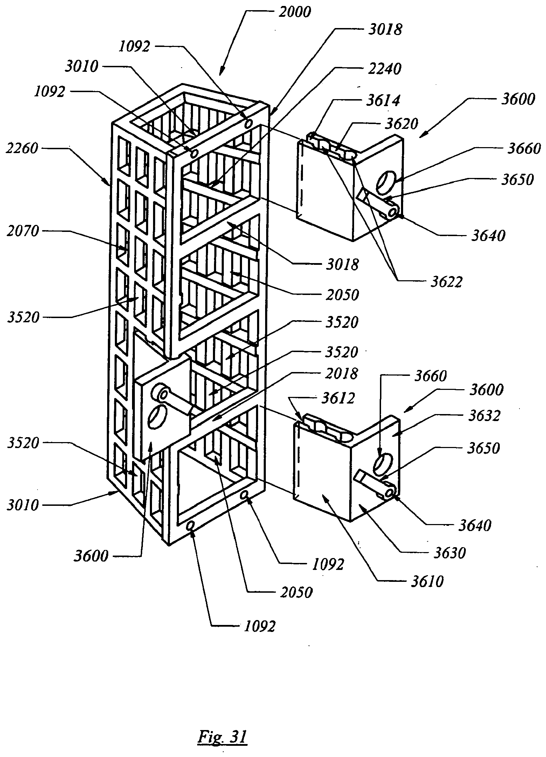

FIG. 31 is an exploded view of cage (2000) and a plurality of attachable lateral brakes (3600). When connected to cage (2000), connectable lateral brakes (3600) extend laterally beyond lateral supports (2240 and 2260), respectively, of cage (2000). Connectable lateral brake (3600) has first wall (3610) and second wall (3630) that is generally perpendicular to first wall (3610). First wall (3610) has an open side (3612) in communication with channel (3614) that extends through the length of first wall (3610). Channel (3614) has a first width (3620) that is narrower than universal posts (2050, 2070) and a second width (3622) that is greater than universal posts (2050, 2070) of first lateral support (2240) or second lateral support (2260) of cage (2000). Second width (3622) locks about the perimeters of universal posts (2050, 2070) to secure first wall (3610) to cage (2000). Second wall (3630) is provided with hub (3640) attached to outward side (3632) of second wall (3630). Rotatable safety (3650) is attached to hub (3640) and is capable of covering at least a part of the circumference of bore (3660) for preventing a fastener (not shown in this view) from backing out of bore (3660).

Regarding the preferred best modes disclosed and enabled in this specification, with respect to the various embodiments of bars, bores, brakes, end caps, grips, inferior dividers, superior dividers, inferior plates and superior plates that create numerous embodiments of the current invention, where engineering parameters require, the bars, bores, brakes, end caps, grips, inferior dividers, superior dividers, inferior plates and superior plates portrayed in FIGS. 1 -30 can be utilized with cages that have generally horizontal dividers or with cages that have a generally horizontal divider and one or more dividers that diverge toward the generally horizontal divider.

Having disclosed the spinal implant, Applicant now prays respectfully that a patent be granted for its invention in accordance with the scope of the claims appended hereto.

## Claims

1. A cage (2000) capable of implantation into a cavity surgically created between a superior spinal region and an inferior spinal region of a mammal; said cage (2000) comprising:
a) an inward support (2200);
b) an outward support (2220);
c) a first lateral support (2240);
d) a second lateral support (2260),
e) wherein said inward support (2200) and said outward support (2220) each are joined with said first lateral support (2240) and said second lateral support (2260) to form said cage (2000) such that said outward support (2220) is of greater dimensions than said inward support (2200);
f) said supports (2200, 2220, 2240, 2260) providing a series of trapezoidal dividers (2010, 3010) comprising superior trapezoidal dividers (3010) and inferior trapezoidal dividers (3010), wherein each trapezoidal divider (2010, 3010) includes an aperture and four sides with two of said sides being of equal length, wherein said trapezoidal divider's (2010, 3010) four sides create said aperture; and
g) more than one first pairs of lateral extensions (2080, 2082, 2102) contacting said outward support (2220) and extending laterally beyond said first lateral support (2240) and more than one second pairs of lateral extensions (2080, 2082, 2102) contacting said outward support (2220) and extending laterally beyond said second lateral support (2260), wherein each pair of lateral extensions (2080, 2082, 2102) creates a grip comprising two corresponding parallel lateral extensions (2080, 2082, 2102) with a gap therebetween, **characterised in that** said gap is small compared to the distances between each said grip of said cage (2000).

2. The cage (2000) of claim 1, wherein said first pairs of lateral extensions (2080, 2082, 2102) are provided with one or more bores (2090, 2098).

3. The cage (2000) of one of the claims 1 or 2, wherein said second pairs of lateral extensions (2080, 2082, 2102) are provided with one or more bores (2090, 2098).

4. The cage (2000) of one of the claims 1 to 3, further comprising one or more bars (2100) extending between and connecting with lateral extensions (2102) of said first pair of lateral extensions and lateral extensions (2102) of said second pair of lateral extensions.

5. The cage (2000) of one of the claims 1 to 4, wherein:
a) said inward support (2200) comprises:
i) a first corner (2030) common with said first lateral support (2240); and
ii) a second corner (2040) common with said second lateral support (2260); and
b) said outward support (2220) comprises a border common with said first lateral support (2240) and said second lateral support (2260).

6. The cage (2000) of one of the claims 1 to 5, wherein said inward support (2200) and said lateral supports (2240, 2260) further comprise one or more posts (2050, 2070, 2072) and one or more ties (2074).

7. The cage (2000) of one of the claims 1 to 6, wherein:
a) a superior divider (2010, 3010) or an inferior divider (2010, 3010) or both superior and inferior dividers (2010, 3010) of said series of trapezoidal dividers include one or more bores (1092, 2090) for receiving one or more fasteners;
and/or
b) a superior divider (2010, 3010) of said series of trapezoidal dividers further comprises a first plate (1100) extending outwardly from said superior divider (2010, 3010).

8. The cage (2000) of one of the claims 1 to 6, wherein:
a) a superior divider (2010, 3010) or an inferior divider (2010,3010) or both superior and inferior dividers (2010, 3010) of said series of trapezoidal dividers include one or more bores (1092, 2090) for receiving one or more fasteners;
and/or
b) a superior divider (2010, 3010) of said series of trapezoidal dividers further comprises a first plate (1100) extending outwardly from said superior divider (2010, 3010) and an inferior divider (2010, 3010) further comprises a second plate (1200) extending outwardly from said inferior divider (2010, 3010).

9. The cage (2000) of one of the claims 1 to 6, wherein:
a) a superior divider (2010, 3010) or an inferior divider (2010,3010) or both superior and inferior dividers (2010, 3010) of said series of trapezoidal dividers include one or more bores (1092, 2090) for receiving one or more fasteners;
and/or
b) a superior divider (2010, 3010) of said series of trapezoidal dividers further comprises a first plate (1100) extending outwardly from said superior divider (2010, 3010), wherein said first plate (1100) is provided with one or more bores (1102).

10. The cage (2000) of one of the claims 1 to 6, wherein:
a) a superior divider (2010, 3010) or an inferior divider (2010,3010) or both superior and inferior dividers (2010, 3010) of said series of trapezoidal dividers include one or more bores (1092, 2090) for receiving one or more fasteners;
and/or
b) a superior divider (2010, 3010) of said series of trapezoidal dividers further comprises a first plate (1100) extending outwardly from said superior divider (2010, 3010) and an inferior divider (2010, 3010) further comprises a second plate (1200) extending outwardly from said inferior divider (2010, 3010), wherein said first plate (1100) and/or said second plate (1200) are provided with one or more bores (1102, 1202).

11. The cage (2000) of one of the claims 8 to 10, wherein:
a) said bores (1092, 1102, 2090) of said superior trapezoidal divider (1100, 2010, 3010) are angled upward relative to outward edges of said superior trapezoidal divider (1100, 2010, 3010);
and/or
b) said bores (1092, 1202, 2090) of said inferior trapezoidal divider (1200, 2010, 3010) are angled downward relative to outward edges of said superior trapezoidal divider (1100, 2010, 3010);
and/or
c) said bores (2090, 2098) of said first pairs of lateral extensions (2080, 2082, 2021) proximate said superior trapezoidal divider (1100, 2010, 3010) are angled upward relative to outward edges of said grips;
and/or
d) said bores (2090, 2098) of said second pairs of lateral extensions (2080, 2082, 2021) proximate said superior trapezoidal divider (1100, 2010, 3010) are angled upward relative to outward edges of said grips;
and/or
e) said bores (2090, 2098) of said first pairs of lateral extensions (2080, 2082, 2102) proximate said inferior trapezoidal divider (1200, 2010, 3010) are angled downward relative to outward edges of said grips;
and/or
f) said bores (2090, 2098) of said second pairs of lateral extensions (2080, 2082, 2102) proximate said inferior trapezoidal divider (1200, 2010, 3010) are angled downward relative to outward edges of said grips.

12. The cage (2000) of one of the claims 1 to 11, wherein one or more end caps (3900) are connected to said cage (2000) and wherein at least one of said end caps (3900) comprises:
a) a bridge (3910); and
b) a coupler (3950) comprising a frontal plate (3954) including at least one hole (3956) for receiving a fastener.

13. The cage (2000) of claim 12, wherein said at least one hole (3956) of said coupler (3950) is proximate to said superior trapezoidal divider (1100, 2010, 3010) and is angled upwardly, or is proximate to said inferior trapezoidal divider (1200, 2010, 3010) and is angled downwardly.

## Patentansprüche

1. Käfig (2000) zur Implantation in eine Kavität, die zwischen einem oberen Wirbelsäulenbereich und einem unteren Wirbelsäulenbereich eines Säugetiers chirurgisch erstellt wird; wobei der Käfig (2000) Folgendes umfasst:
a) einen nach innen gerichteten Träger (2200);
b) einen nach außen gerichteten Träger (2220);
c) einen ersten seitlichen Träger (2240);
d) einen zweiten seitlichen Träger (2260),
e) wobei der nach innen gerichtete Träger (2200) und der nach außen gerichtete Träger (2220) jeweils mit dem ersten seitlichen Träger (2240) und dem zweiten seitlichen Träger (2260) zusammengefügt sind, um den Käfig (2000) zu bilden, so dass der nach außen gerichtete Träger (2220) größere Abmessungen als der nach innen gerichtete Träger (2200) aufweist;
f) wobei die Träger (2200, 2220, 2240, 2260) eine Reihe von trapezförmigen Trennelementen (2010, 3010) bereitstellen, die obere trapezförmige Trennelemente (3010) und untere trapezförmige Trennelemente (3010) umfassen, wobei jedes trapezförmige Trennelement (2010, 3010) eine Öffnung und vier Seiten umfasst, wobei zwei der Seiten gleich lang sind, wobei die vier Seiten des trapezförmigen Trennelements (2010, 3010) die Öffnung bilden; und
g) wobei mehr als ein erstes Paar von seitlichen Verlängerungen (2080, 2082, 2102) den nach außen gerichteten Träger (2220) berühren und sich seitlich über den ersten seitlichen Träger (2240) hinaus erstrecken, und mehr als ein zweites Paar von seitlichen Verlängerungen (2080, 2082, 2102) den nach außen gerichteten Träger (2220) berühren und sich seitlich über den zweiten seitlichen Träger (2260) hinaus erstrecken, wobei jedes Paar von seitlichen Verlängerungen (2080, 2082, 2102) einen Griff ausbildet, der zwei dazugehörige parallele seitliche Verlängerungen (2080, 2082, 2102) mit einer Lücke dazwischen umfasst, **dadurch gekennzeichnet, dass** die Lücke im Vergleich zu den Abständen zwischen jedem Griff des Käfigs (2000) klein ist.

2. Käfig (2000) nach Anspruch 1, wobei die ersten Paare von seitlichen Verlängerungen (2080, 2082, 2102) mit einer oder mehreren Bohrungen (2090, 2098) versehen sind.

3. Käfig (2000) nach einem der Ansprüche 1 oder 2, wobei die zweiten Paare von seitlichen Verlängerungen (2080, 2082, 2102) mit einer oder mehreren Bohrungen (2090, 2098) versehen sind.

4. Käfig (2000) nach einem der Ansprüche 1 bis 3, ferner umfassend einen oder mehrere Stäbe (2100), der bzw. die sich zwischen den seitlichen Verlängerungen (2102) des ersten Paars von seitlichen Verlängerungen und den seitlichen Verlängerungen (2102) des zweiten Paars von seitlichen Verlängerungen erstreckt bzw. erstrecken und damit verbunden ist bzw. sind.

5. Käfig (2000) nach einem der Ansprüche 1 bis 4, wobei:
a) der nach innen gerichtete Träger (2200) Folgendes umfasst:
i) eine erste Ecke (2030), die mit dem ersten seitlichen Träger (2240) gemeinsam ist; und
ii) eine zweite Ecke (2040), die mit dem zweiten seitlichen Träger (2260) gemeinsam ist; und
b) der nach außen gerichtete Träger (2220) eine Grenze umfasst, die mit dem ersten seitlichen Träger (2240) und dem zweiten seitlichen Träger (2260) gemeinsam ist.

6. Käfig (2000) nach einem der Ansprüche 1 bis 5, wobei der nach innen gerichtete Träger (2200) und die seitlichen Träger (2240, 2260) ferner eine oder mehrere Stützen (2050, 2070, 2072) und eine oder mehrere Verbindungsteile (2074) umfassen.

7. Käfig (2000) nach einem der Ansprüche 1 bis 6, wobei:
a) ein oberes Trennelement (2010, 3010) oder ein unteres Trennelement (2010, 3010) oder sowohl das obere als auch das untere Trennelement (2010, 3010) der Reihe von trapezförmigen Trennelementen eine oder mehrere Bohrungen (1092, 2090) umfassen, um ein oder mehrere Befestigungselemente aufzunehmen;
und/oder
b) ein oberes Trennelement (2010, 3010) der Reihe von trapezförmigen Trennelementen ferner eine erste Platte (1100) umfasst, die sich von dem oberen Trennelement (2010, 3010) aus nach außen erstreckt.

8. Käfig (2000) nach einem der Ansprüche 1 bis 6, wobei:
a) ein oberes Trennelement (2010, 3010) oder ein unteres Trennelement (2010, 3010) oder sowohl das obere als auch das untere Trennelement (2010, 3010) der Reihe von trapezförmigen Trennelementen eine oder mehrere Bohrungen (1092, 2090) umfassen, um ein oder mehrere Befestigungselemente aufzunehmen;
und/oder
b) ein oberes Trennelement (2010, 3010) der Reihe von trapezförmigen Trennelementen ferner eine erste Platte (1100) umfasst, die sich von dem oberen Trennelement (2010, 3010) aus nach außen erstreckt, und ein unteres Trennelement (2010, 3010) ferner eine zweite Platte (1200) umfasst, die sich von dem unteren Trennelement (2010, 3010) aus nach außen erstreckt.

9. Käfig (2000) nach einem der Ansprüche 1 bis 6, wobei:
a) ein oberes Trennelement (2010, 3010) oder ein unteres Trennelement (2010, 3010) oder sowohl das obere als auch das untere Trennelement (2010, 3010) der Reihe von trapezförmigen Trennelementen eine oder mehrere Bohrungen (1092, 2090) umfassen, um ein oder mehrere Befestigungsmittel aufzunehmen;
und/oder
b) ein oberes Trennelement (2010, 3010) der Reihe von trapezförmigen Trennelementen ferner eine erste Platte (1100) umfasst, die sich von dem oberen Trennelement (2010, 3010) aus nach außen erstreckt, wobei die erste Platte (1100) mit einer oder mehreren Bohrungen (1102) versehen ist.

10. Käfig (2000) nach einem der Ansprüche 1 bis 6, wobei:
a) ein oberes Trennelement (2010, 3010) oder ein unteres Trennelement (2010, 3010) oder sowohl das obere als auch das untere Trennelement (2010, 3010) der Reihe von trapezförmigen Trennelementen eine oder mehrere Bohrungen (1092, 2090) umfassen, um ein oder mehrere Befestigungsmittel aufzunehmen;
und/oder
b) ein oberes Trennelement (2010, 3010) der Reihe von trapezförmigen Trennelementen ferner eine erste Platte (1100) umfasst, die sich von dem oberen Trennelement (2010, 3010) aus nach außen erstreckt, und ein unteres Trennelement (2010, 3010) ferner eine zweite Platte (1200) umfasst, die sich von dem unteren Trennelement (2010, 3010) aus nach außen erstreckt, wobei die erste Platte (1100) und/oder die zweite Platte (1200) mit einer oder mehreren Bohrungen (1102, 1202) versehen ist bzw. sind.

11. Käfig (2000) nach einem der Ansprüche 8 bis 10, wobei:
a) die Bohrungen (1092, 1102, 2090) des oberen trapezförmigen Trennelements (1100, 2010, 3010) in Bezug auf nach außen gerichtete Kanten des oberen trapezförmigen Trennelements (1100, 2010, 3010) nach oben angewinkelt sind;
und/oder
b) die Bohrungen (1092, 1202, 2090) des unteren trapezförmigen Trennelements (1200, 2010, 3010) in Bezug auf nach außen gerichtete Kanten des oberen trapezförmigen Trennelements (1100, 2010, 3010) nach unten angewinkelt sind;
und/oder
c) die Bohrungen (2090, 2098) der ersten Paare von seitlichen Verlängerungen (2080, 2082, 2021) in der Nähe des oberen trapezförmigen Trennelements (1100, 2010, 3010) in Bezug auf nach außen gerichtete Kanten der Griffe nach oben angewinkelt sind; und/oder
d) die Bohrungen (2090, 2098) der zweiten Paare von seitlichen Verlängerungen (2080, 2082, 2021) in der Nähe des oberen trapezförmigen Trennelements (1100, 2010, 3010) in Bezug auf nach außen gerichtete Kanten der Griffe nach oben angewinkelt sind;
und/oder
e) die Bohrungen (2090, 2098) der ersten Paare von seitlichen Verlängerungen (2080, 2082, 2102) in der Nähe des unteren trapezförmigen Trennelements (1200, 2010, 3010) in Bezug auf nach außen gerichtete Kanten der Griffe nach unten angewinkelt sind;
und/oder
f) die Bohrungen (2090, 2098) der zweiten Paare von seitlichen Verlängerungen (2080, 2082, 2102) in der Nähe des unteren trapezförmigen Trennelements (1200, 2010, 3010) in Bezug auf nach außen gerichtete Kanten der Griffe nach unten angewinkelt sind.

12. Käfig (2000) nach einem der Ansprüche 1 bis 11, wobei eine oder mehrere Endkappen (3900) mit dem Käfig (2000) verbunden ist bzw. sind, und wobei mindestens eine der Endkappen (3900) Folgendes umfasst:
a) eine Brücke (3910); und
b) einen Koppler (3950), der eine Vorderplatte (3954) umfasst, die mindestens ein Loch (3956) umfasst, um ein Befestigungselement aufzunehmen.

13. Käfig (2000) nach Anspruch 12, wobei das mindestens eine Loch (3956) des Kopplers (3950) in der Nähe des oberen trapezförmigen Trennelements (1100, 2010, 3010) liegt und nach oben angewinkelt ist oder sich in der Nähe des unteren trapezförmigen Trennelements (1200, 2010, 3010) befindet und nach unten angewinkelt ist.

## Revendications

1. Cage (2000) en mesure d'être implantée jusque dans une cavité créée de façon chirurgicale entre une région spinale supérieure et une région spinale inférieure d'un mammifère, ladite cage (2000) comprenant :
a) un support intérieur (2200) ;
b) un support extérieur (2220) ;
c) un premier support latéral (2240) ;
d) un second support latéral (2260) ;
e) dans lequel ledit support intérieur (2200) et ledit support extérieur (2220) sont chacun joints avec ledit premier support latéral (2240) et ledit second support latéral (2260) pour former ladite cage (2000) de telle façon que ledit support extérieur (2220) a de plus grandes dimensions que ledit support intérieur (2200) ;
f) lesdits supports (2200, 2220, 2240, 2260) fournissant une série de diviseurs trapézoïdaux (2010, 3010) comprenant des diviseurs trapézoïdaux supérieurs (3010) et des diviseurs trapézoïdaux inférieurs (3010), dans lequel chaque diviseur trapézoïdal (2010, 3010) comprend une ouverture et quatre côtés avec deux desdits côtés étant de longueur égale, dans lequel lesdits quatre côtés du diviseur trapézoïdal (2010, 3010) créent ladite ouverture ; et
g) plus d'une première paire d'extensions latérales (2080, 2082, 2102) étant en contact avec ledit support extérieur (2220) et s'étendant latéralement au-delà dudit premier support latéral (2240) et plus d'une seconde paire d'extensions latérales (2080, 2082, 2102) étant en contact avec ledit support extérieur (2220) et s'étendant latéralement au-delà dudit second support latéral (2260), dans laquelle chaque paire d'extensions latérales (2080, 2082, 2102) crée une prise comprenant deux extensions latérales (2080, 2082, 2102) parallèles correspondantes avec un espace entre elles, **caractérisée en ce que** ledit espace est petit comparé aux distances entre chaque dite prise de ladite cage (2000).

2. Cage (2000) selon la revendication 1, dans lequel lesdites premières paires d'extensions latérales (2080, 2082, 2102) sont dotées d'un ou plusieurs alésage(s) (2090, 2098).

3. Cage (2000) selon l'une des revendications 1 ou 2, dans lequel lesdites secondes paires d'extensions latérales (2080, 2082, 2102) sont dotées d'un ou plusieurs alésage(s) (2090, 2098).

4. Cage (2000) selon l'une des revendications 1 à 3, comprenant en outre une ou plusieurs barre(s) (2100) s'étendant et se connectant à des extensions latérales (2102) de ladite première paire d'extensions latérales et à des extensions latérales (2102) de ladite seconde paire d'extensions latérales.

5. Cage (2000) selon l'une des revendications 1 à 4, dans laquelle :
a) ledit support intérieur (2200) comprend :
i) un premier coin (2030) commun avec ledit premier support latéral (2240) ; et
ii) un second coin (2040) commun avec ledit second support latéral (2260) ; et
b) ledit support extérieur (2220) comprend une frontière commune avec ledit premier support latéral (2240) et ledit second support latéral (2260).

6. Cage (2000) selon l'une des revendications 1 à 5, dans laquelle ledit support intérieur (2200) et lesdits supports latéraux (2240, 2260) comprennent en outre un ou plusieurs pilier(s) (2050, 2070, 2072) et une ou plusieurs traverse(s) (2074).

7. Cage (2000) selon l'une des revendications 1 à 6, dans laquelle :
a) un diviseur supérieur (2010, 3010) ou un diviseur inférieur (2010, 3010) ou les deux diviseurs supérieur et inférieur (2010, 3010) de ladite série de diviseurs trapézoïdaux inclu(en)t un ou plusieurs alésage (s) (1092, 2090) pour recevoir une ou plusieurs fixation(s) ;
et/ou
b) un diviseur supérieur (2010, 3010) de ladite série de diviseurs trapézoïdaux comprend en outre une première plaque (1100) s'étendant vers l'extérieur dudit diviseur supérieur (2010, 3010).

8. Cage (2000) selon l'une des revendications 1 à 6, dans laquelle :
a) un diviseur supérieur (2010, 3010) ou un diviseur inférieur (2010, 3010) ou les deux diviseurs supérieur et inférieur (2010, 3010) de ladite série de diviseurs trapézoïdaux inclu(en)t un ou plusieurs alésage(s) (1092, 2090) pour recevoir une ou plusieurs fixation(s) ;
et/ou
b) un diviseur supérieur (2010, 3010) de ladite série de diviseurs trapézoïdaux comprend en outre une première plaque (1100) s'étendant vers l'extérieur dudit diviseur supérieur (2010, 3010) et un diviseur inférieur (2010, 3010) comprend en outre une seconde plaque (1200) s'étendant vers l'extérieur dudit diviseur inférieur (2010, 3010).

9. Cage (2000) selon l'une des revendications 1 à 6, dans laquelle :
a) un diviseur supérieur (2010, 3010) ou un diviseur inférieur (2010, 3010) ou les deux diviseurs supérieur et inférieur (2010, 3010) de ladite série de diviseurs trapézoïdaux inclu(en)t un ou plusieurs alésage(s) (1092, 2090) pour recevoir une ou plusieurs fixation(s) ;
et/ou
b) un diviseur supérieur (2010, 3010) de ladite série de diviseurs trapézoïdaux comprend en outre une première plaque (1100) s'étendant vers l'extérieur dudit diviseur supérieur (2010, 3010), dans lequel ladite première plaque (1100) est dotée d'un ou plusieurs alésage(s) (1102).

10. Cage (2000) selon l'une des revendications 1 à 6, dans laquelle :
a) un diviseur supérieur (2010, 3010) ou un diviseur inférieur (2010, 3010) ou les deux diviseurs supérieur et inférieur (2010, 3010) de ladite série de diviseurs trapézoïdaux inclu(en)t un ou plusieurs alésage (s) (1092, 2090) pour recevoir une ou plusieurs fixation(s) ;
et/ou
b) un diviseur supérieur (2010, 3010) de ladite série de diviseurs trapézoïdaux comprend une première plaque (1100) s'étendant vers l'extérieur dudit diviseur supérieur (2010, 3010) et un diviseur inférieur (2010, 3010) comprend en outre une seconde plaque (1200) s'étendant vers l'extérieur dudit diviseur inférieur (2010, 3010), dans lequel ladite première plaque (1100) et/ou ladite seconde plaque (1200) sont dotées d'un ou plusieurs alésage(s) (1102, 1202).

11. Cage (2000) selon l'une des revendications 8 à 10, dans laquelle :
a) lesdits alésages (1092, 1102, 2090) dudit diviseur trapézoïdal supérieur (1100, 2010, 3010) sont en angle ascendant par rapport aux bords extérieurs dudit diviseur trapézoïdal supérieur (1100, 2010, 3010) ;
et/ou
b) lesdits alésages (1092, 1202, 2090) dudit diviseur trapézoïdal inférieur (1200, 2010, 3010) sont en angle descendant par rapport aux bords extérieurs dudit diviseur trapézoïdal supérieur (1100, 2010, 3010) ;
et/ou
c) lesdits alésages (2090, 2098) desdites premières paires d'extensions latérales (2080, 2082, 2102) proches dudit diviseur trapézoïdal supérieur (1100, 2010, 3010) sont en angle ascendant par rapport aux bords extérieurs desdites prises ;
et/ou
d) lesdits alésages (2090, 2098) desdites secondes paires d'extensions latérales (2080, 2082, 2102) proches dudit diviseur trapézoïdal supérieur (1100, 2010, 3010) sont en angle ascendant par rapport aux bords extérieurs desdites prises ;
et/ou
e) lesdits alésages (2090, 2098) desdites premières paires d'extensions latérales (2080, 2082, 2102) proches dudit diviseur trapézoïdal inférieur (1200, 2010, 3010) sont en angle descendant par rapport aux bords extérieurs desdites prises ;
et/ou
f) lesdits alésages (2090, 2098) desdites secondes paires d'extensions latérales (2080, 2082, 2102) proches dudit diviseur trapézoïdal inférieur (1200, 2010, 3010) sont en angle descendant par rapport aux bords extérieurs desdites prises.

12. Cage (2000) selon l'une des revendications 1 à 11, dans laquelle un ou plusieurs bouchon(s) d'extrémité (3900) sont connectés à ladite cage (2000) et dans lequel au moins un desdits bouchons d'extrémité (3900) comprend :
a) un pont (3910) ; et
b) un coupleur (3950) comprenant une plaque frontale (3954) incluant au moins un orifice (3956) pour recevoir une fixation.

13. Cage (2000) selon la revendication 12, dans lequel ledit au moins un orifice (3956) dudit coupleur (3950) est proche dudit diviseur trapézoïdal supérieur (1100, 2010, 3010) et est en angle ascendant, ou est proche dudit diviseur trapézoïdal inférieur (1200, 2010, 3010) et est en angle descendant.
